# EUROPÄISCHE PATENTANMELDUNG

(11) **EP 4 039 638 A1**
(43) Veröffentlichungstag der Anmeldung: **10.08.2022**
(21) Anmeldenummer: 21155101.5
(22) Anmeldetag: 03.02.2021
(51) Int. Cl.: C01B 3/16, C01B 3/38, C01B 3/50, C01B 32/80, C07C 209/36, C07C 263/10, C25B 1/04, C25B 1/16, C25B 1/26, C01B 7/04

(54) **VERFAHREN ZUR HERSTELLUNG VON KOHLENMONOXID ALS ROHSTOFF ZUR ISOCYANATHERSTELLUNG MIT VERRINGERTEM CO2 FUSSABDRUCK**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Es wird ein Verfahren zur Herstellung von Kohlenmonoxid beschrieben, das für die Einbettung in ein Herstellungsverfahren für Isocyanate durch Synthese (1) von Phosgen (1a) aus Kohlenmonoxid (9a) und Chlor (12b), Umsetzung (2) von Phosgen (1a) mit Aminen (23) zu Isocyanaten (24) und Chlorwasserstoff (25) vorgesehen ist, in dem Kohlendioxid, Methan und Wasserdampf in einem Reformerprozess (6) unter Zuführung von Wärmeenergie (62) bei einer Temperatur von mindestens 500°C umgesetzt wird, wobei
- die zur Synthese von Kohlenmonoxid dem Reformerprozess (6) zugeführte Wärmeenergie (62) durch mindestens eine Methode bereitgestellt wird, ausgewählt aus (i) der Verbrennung von Brennstoff, der mittels regenerativer Energie erzeugten Wasserstoff enthält, (ii) der Verbrennung von Brennstoff, der Methan aus biologischer Quelle enthält, (iii) Umwandlung von aus regenerativer Energie erzeugter, elektrischer Energie in Wärme; und/oder
- für die Bereitgestellung des der Synthese des vorgenannten Reformerprozesses (6) zugesetzten CO₂ zusätzlich CO₂ aus einer weiteren CO₂-Quelle herangezogen wird.

## Beschreibung

Die Erfindung betrifft ein Verfahren zur emissionsverringerten Herstellung von Kohlenmonoxid als Rohstoff zur Isocyanatherstellung unter Einsatz eines Reformerprozesses, in dem neben Methan zusätzlich Kohlendioxid und Wasserdampf, sowie Wärmeenergie aus regenerativer Energiequelle eingesetzt werden. Das aus dem Reformerprozess erhaltende Gas wird aufbereitet und das gewonnene Kohlenmonoxid beispielsweise für eine Umsetzung mit Chlor zu Phosgen vorgesehen. Aus Phosgen und organischen Aminen können die organischen Isocyanate und diese durch Umsetzung mit Polyolen und/oder Polyestern Polyurethan-Material hergestellt werden.

Polyurethane, nachfolgend auch abgekürzt PU genannt, sind Kunststoffe, die aus der Polyadditionsreaktion von mindestens zwei Hydroxylgruppen enthaltenden Polyolen mit Polyisocyanaten entstehen. Der Einsatz von Diolen und Diisocyanaten führt zu linearen Polyurethanen. Vernetzte Polyurethane können durch Umsetzung von Triisocyanat-Diisocyanat-Gemischen mit Triol-Diol-Gemischen hergestellt werden. Die Eigenschaften von PU können in einem weiten Rahmen variiert werden. Je nach Vernetzungsgrad und/oder eingesetzter Isocyanat- oder OH-Komponente erhält man Duroplaste, Thermoplaste oder Elastomere. Polyurethane werden jedoch auch als Formmassen zum Formpressen, als Gießharze (Isocyanat-Harze), als (textile) elastische Faserstoffe, Polyurethanlacke und als Polyurethanklebstoffe verwendet. Aus Polyurethan lassen sich auch sehr einfach Schaumstoffe herstellen.

Weiche PU-Schaumstoffe werden für sehr viele Zwecke verwendet, vor allem als Polstermaterial z. B. für Möbel und Autositze, als Matratzenschaum, als Teppichrückenmaterial, zur Textilkaschierung, als Reinigungsschwamm oder als Filtermaterial.

PU-Hartschäume werden vor allem zur Wärmedämmung z. B. in Gebäuden, Kühlgeräten, Wärme- und Kältespeichern sowie einigen Rohrsystemen (Kunststoffmantelverbundrohr, flexible Verbundrohre) eingesetzt.

Weitere, relativ neue Anwendungsgebiete für PU-Schäume gibt es im Fahrzeugbau wie Lenkrad, Armauflage, Softbeschichtung von Handgriffen, Innenraumverkleidung, Armaturenbrett, Schalldämmung, Klapperschutz, Abdichtungen, Transparentbeschichtung von Holzdekoren.

Bisher werden wesentliche Komponenten zur Polyurethanherstellung wie das Kohlenmonoxid, Wasserstoff oder der Strom zum Betrieb der Elektrolysen wie der Wasserelektrolyse und der Chlor-Alkali-Elektrolyse aus fossilen Energieträgern hergestellt. So wird konventionell Kohlenmonoxid und Wasserstoff aus Erdgas bzw. aus Kohle mittels Reformingprozessen, und Chlor aus der Elektrolyse mit Strom, der unter Einsatz von fossilen Brennstoffen wie Öl, Kohle oder Erdgas hergestellt wird, gewonnen. Dabei entsteht eine erhebliche Menge an CO₂.

Aufgabe der vorliegenden Erfindung war es, ein nachhaltigeres Verfahren für die Kohlenmonoxidherstellung und letztlich auch für die Isocyanatherstellung, aber auch für die PolyurethanHerstellung zu finden.

Weiterhin sollte die nachhaltigere Lösung im Rahmen einer weiteren Aufgabe der vorliegenden Erfindung ohne großen Mehraufwand in den bestehenden Produktionsprozessen anwendbar und darin implementierbar sein.

Unter "Nachhaltigkeit" eines Verfahrens versteht der Fachmann in Anwendung der durch die UN geprägte Nachhatigkeits-Definition ("sustainable development") gemäß Brundtlandbericht der "Weltkommission für Umwelt und Entwicklung", dass durch die Ausführung des Verfahrens in der Gegenwart ein möglichst geringer bis gar kein Beitrag geleistet wird, dass künftige Generationen der Menschheit ihre eigenen Bedürfnisse nicht mehr befriedigen können, insbesondere Bedürfnisse mit Blick auf die Nutzung von Ressourcen wie z.B. fossiler Rohstoffe und insbesondere mit Blick auf die Schonung des Lebensraumes, wie z.B. den Schutz der Erdatmosphäre. Die Erfindung hat somit zur Aufgabe, die Produktion von Kohlenmonoxid, aber auch von Isocyanat und Polyurethan nachhaltiger als die aus dem Stand der Technik bekannten Produktionsmethoden zu gestalten. Der Beitrag der Produktion von Isocyanat und Polyurethan zu einer sinkenden Befriedigung der Bedürfnisse zukünftiger Generationen soll vermindert bis vermieden werden.

Eine Aufgabe der Erfindung ist es also, den Einsatz von fossilen Rohstoffen zur Bereitstellung von Energie für die besagte Kohlenmonoxidproduktion zu vermindern und gegebenenfalls auch den Einsatz von fossilen Rohstoffen als Edukt für die Produktion von in der Isocyanatproduktion verwendbarem Kohlenmonoxid zu reduzieren. Dadurch soll die Kohlendioxid-Bilanz (Carbon Foot Print) der Produktion von Rohstoffen für die Isocyanat- und für die PU-Herstellung zum Schutz der Erdatmosphäre weiter verbessert werden.

Zur Lösung mindestens einer der vorgenannten Aufgaben eignet sich als erster Gegenstand der Erfindung ein Verfahren für die Herstellung von Kohlenmonoxid aus Methan, Wasserdampf und CO₂ für die Darstellung von Phosgen zur Synthese von organischem Isocyanat, enthaltend zumindest die Schritte
Synthese von Kohlenmonoxid in einem Reformerprozess, worin Methan und Wasserdampf unter Zusatz von mindestens CO₂,
unter Zuführung von Wärmeenergie bei einer Temperatur von mindestens 500°C zu Kohlenmonoxid-haltigem Produktgas umgesetzt wird,
Reinigung des aus vorgenannter Synthese erhaltenen Kohlenmonoxid-haltigen Produktgases, mindestens durch Abtrennung von CO₂ und gegebenenfalls zusätzlich durch mindestens eine Abtrennung ausgewählt aus der Abtrennung von Wasser, der Abtrennung von Wasserstoff oder einer Kombination davon, unter Erhalt von Kohlenmonoxid;
Bereitstellung von CO₂ für den besagten Zusatz zum vorgenannten Reformerprozess zumindest aus besagter Abtrennung von CO₂ des vorgenannten Reinigungsschritts,
mit der Maßgabe, dass
   i) die zur Synthese von Kohlenmonoxid dem Reformerprozess zugeführte Wärmeenergie durch mindestens eine Methode bereitgestellt wird, ausgewählt aus (i) der Verbrennung von Brennstoff, der mittels regenerativer Energie erzeugten Wasserstoff enthält, (ii) der Verbrennung von Brennstoff, der Methan aus biologischer Quelle enthält, (iii) Umwandlung von aus regenerativer Energie erzeugter, elektrischer Energie in Wärme; oder
   ii) für die Bereitgestellung des der Synthese des vorgenannten Reformerprozesses zugesetzten CO₂ zusätzlich CO₂ aus einer weiteren CO₂-Quelle herangezogen wird; oder
   iii) eine Kombination aus i) und ii) gewählt wird.

Der zur Synthese von Kohlenmonoxid durchgeführte Reformerprozess wird beispielsweise in einem Reaktor durchgeführt, dem sogenannten Reformer. Dieser Reformer besitzt bevorzugt ein Heizelement als eine Vorrichtung zur Zuführung von Wärmeenergie, sowie mindestens einen Einlass, durch den Methan, Wasserdampf und CO₂ in den Reformer eingebracht werden können, und mindestens einen Auslass für das Kohlenmonoxid-haltige Produktgas.

Ein organischer Stoff ist eine chemische Verbindung, die mindestens eine kovalente Kohlenstoff-Wasserstoff-Bindung im Molekül enthält. Ein organisches Isocyanat ist demnach ein organischer Stoff, der als chemische Verbindung mindestens eine Isocyanatgruppe und mindestens eine kovalente Kohlenstoff-Wasserstoff-Bindung im Molekül enthält. Ein organisches Amin ist *mutatis mutandis* definiert.

Durch den besagten Reformerprozess werden im Rahmen der Maßgabe die eingesetzten Rohstoffe wie beispielsweise Methan aus Erdgas oder aus Bio-Gas, Wasserdampf und CO₂ zur Reaktion gebracht und zu einem Produktgasgemisch enthaltend Wasserstoff und CO und gegebenenfalls Nebenprodukte umgesetzt. Als Nebenprodukte gelten insbesondere niedere Kohlenwasserstoffe. Unter "niederen Kohlenwasserstoffen" werden erfindungsgemäß Kohlenwasserstoffe mit 1 bis 8 Kohlenstoffatomen verstanden. Im Produktgasgemisch wird sich auch nicht umgesetztes CO₂ und Wasserdampf befinden.

Das in dem erfindungsgemäßen Verfahren im besagten Reformerprozess als Edukt zur Synthese eingesetzte Methan wird in einer bevorzugten Ausführungsform aus einer Methan-Quelle bereitgestellt, ausgewählt aus Methan aus fossiler Quelle (beispielsweise Erdgas), Methan aus biologischer Quelle oder Gemischen daraus, insbesondere Methan aus biologischer Quelle. Wird Methan aus biologischer Quelle ausgewählt ist es wiederum im Rahmen einer weiteren Ausführungsform des Verfahrens besonders bevorzugt, wenn die der Synthese von Kohlenmonoxid zugeführte Wärmeenergie durch mindestens eine Methode bereitgestellt wird, ausgewählt aus (i) der Verbrennung von Brennstoff, der mittels regenerativer Energie erzeugten Wasserstoff enthält, (ii) der Verbrennung von Brennstoff, der Methan aus biologischer Quelle enthält, (iii) Umwandlung von aus regenerativer Energie erzeugter, elektrischer Energie in Wärme.

Unter "Methan aus biologischer Quelle" versteht der Fachmann in Abgrenzung zu fossilem Methan solches Methan, welches technisch durch Methan-Gärung aus Biomasse gewonnen wird. Die Methan-Gärung ist bekanntermaßen der anaerobe Abbau organischer Stoffe durch Mikroorganismen. Methan aus biologischer Quelle wird beispielsweise in Biogasanlagen hergestellt, worin sowohl organische Abfälle als auch nachwachsende Rohstoffe entsprechend vergoren werden.

Der im besagten Reformerprozess zur Synthese von Kohlenmonoxid eingesetzte Wasserdampf wird beispielsweise aus entmineralisiertem Wasser erhalten. Dazu wird beispielsweise Wasser nach herkömmlichen Verfahren entmineralisiert und, wenn möglich, das erhaltene entmineralisierte Wasser mit dem Kondensat aus der vorgenannten Reinigung durch Abtrennung von Wasser aus dem Kohlenmonoxid-haltigen Produktgas vereinigt. Das Wasser kann sodann, z.B. unter Ausnutzung der Wärme des erhaltenen Kohlenmonoxid-haltigen Produktgases, vorgeheizt und einem Entgaser zugeführt werden. In dem Entgaser werden dabei die im Wasser gelösten Gase, wie z.B. Sauerstoff, entfernt. Diese Entfernung kann beispielsweise durch herkömmliche Abreicherungsmethoden, wie z.B. dem Strippen (beispielsweise durch Einleitung von Wasserdampf) oder der Zufügung von absorbierenden Chemikalien, erzielt werden. Nach der Entgasung wird das entgaste Wasser, z.B. unter Ausnutzung der Wärme des erhaltenen Kohlenmonoxid-haltigen Produktgases, nochmals erwärmt und im Anschluss daran in die Dampftrommel übergeführt. Dort wird es in Wasserdampf, beispielsweise überhitzten Wasserdampf, umgewandelt, der dem Reformerprozess des erfindungsgemäßen Verfahrens zugeführt werden kann.

Die Zuführung von CO₂ zum besagten Reformerprozess zur Synthese von Kohlenmonoxid muss erfindungsgemäß zumindest mit CO₂ aus besagter Abtrennung von CO₂ des vorgenannten Reinigungsschritts erfolgen.

Zugeführtes CO₂ kann aber im Rahmen der besagten Maßgabe zusätzlich aus einer weiteren CO₂-Quelle stammen, beispielsweise das bei der Bereitstellung von Wärmeenergie emittierte CO₂ aus der Brennkammer des Heizelementes, oder CO₂ aus einer externen CO₂-Quelle.

Als eine weitere CO₂-Quelle dient bevorzugt mindestens eine externe CO₂-Quelle, die CO₂ beiträgt, welches nicht durch das erfindungsgemäße Verfahren emittiert wird. Eine externe CO₂ Quelle wäre z.B. das CO₂, das bei einer Zementherstellung, bei einer H₂-Herstellung für die Ammoniaksynthese anfällt, bei Verbrennung von Brennstoffen (z.B. einer Abfallverbrennung) im Abgas entsteht, oder CO₂, das aus der Luft gewonnenen wird. Dieses CO₂ aus einer externen CO₂-Quelle wird im Rahmen einer bevorzugten Ausführungsform des erfindungsgemäßen Verfahrens durch Absorption eines CO₂ -Anteils aus (i) Prozessgasen oder Abgasen, die aus mindestens einem Prozess ausgewählt aus Zementherstellung, H₂-Herstellung, Verbrennung ausgewählt wird und/oder (ii) aus Luft durch einleiten in Alkalilauge, zum Beispiel Kalilauge, erfolgen. Hierbei bildet sich Kaliumhydrogencarbonat, welches anschließend wieder zu CO₂ und Kalilauge thermisch zersetzt werden kann. Das dabei freigesetzte CO₂ wird dann dem erfindungsgemäßen Reformerprozess zur Synthese von Kohlenmonoxid zugeführt. Zur Bereitstellung der für die CO₂ Freisetzung notwendigen Wärmeenergie kann beispielsweise die erzeugte Wärmeenergie aus dem besagten Reformerprozess eingesetzt werden.

Ggf. sind weitere Reinigungsschritte notwendig, um eine ausreichende Reinheit des CO₂ für den besagten Reformerprozess zu erzeugen. Das gereinigte CO₂ wird anschließend dem besagten Reformerprozess zugeführt.

Durch den Verbrauch von CO₂ und/oder durch die Nutzung von regenerativer Energie für die Bereitstellung von Brennstoffen bzw. zur elektrischen Beheizung des Reformers und/oder durch den Einsatz von Methan aus biologischer Quelle als Brennstoff können Kohlenmonoxid und vorzugsweise daraus Phosgen und Isocyanate mit weiter verbesserter Nachhaltigkeit hergestellt werden. Der Anteil von fossilem Kohlenstoff im Isocyanat und die Emissionen bei der Herstellung von Kohlenmonoxid im Reformerprozess werden durch das erfindungsgemäße Verfahren deutlich vermindert.

Der für die Synthese von Kohlenmonoxid notwendige Energieeintrag wird in üblichen Reformerprozessen überwiegend durch Nutzung von fossilen Brennstoffen, wie z.B. Erdgas, als Energiequelle erzeugt. Dem erfindungsgemäßen Reformerprozess zur Synthese von Kohlenmonoxid wird für die Einstellung der notwendigen Temperatur zwingend Wärmeenergie zugeführt, die im Rahmen der besagten Maßgabe abweichend davon durch mindestens eine Methode bereitgestellt wird, ausgewählt aus (i) der Verbrennung von Brennstoff, der mittels regenerativer Energie erzeugten Wasserstoff (gasförmiges H₂) enthält, (ii) der Verbrennung von Brennstoff, der Methan aus biologischer Quelle enthält oder (iii) der Umwandlung von aus regenerativer Energie erzeugter, elektrischer Energie in Wärme. Dabei ist es wiederum besonders bevorzugt, wenn als regenerative Energie wahlweise Windkraft, Sonnenenergie, Wasserkraft oder Mischungen daraus, eingesetzt wird. Unter "regenerativer Energie" versteht der Fachmann Energie aus einer Energiequelle, die sich nicht erschöpft, wie z.B. Windenergie, Wasserenergie oder Sonnenenergie.

Die nach erfindungsgemäßen Methoden hergestellte Wärmeenergie für die Synthese des Reformerprozesses kann für die Methoden (i) oder (ii) zusätzlich als Brennstoff fossile Brennstoffe einsetzen. Dies kann beispielsweise im Gemisch mit den in Methode (i) bzw. (ii) genannten Brennstoffen erfolgen, allerdings wird im Rahmen einer erfindungsgemäß bevorzugten Ausführungsform bei Anwendung der Verbrennung von Brennstoffen derart vorgegangen, dass der eingesetzte Brennstoff bezogen auf sein Gesamtgewicht mindestens 20 Gew.-%, besonders bevorzugt mehr als 50 Gew.-%, weiter bevorzugt mindestens 60 Gew.-%, weiter bevorzugt mindestens 80 Gew.-%, am bevorzugtesten mindestens 95 bis 100 Gew.-%, Brennstoff enthält, ausgewählt aus mittels regenerativer Energie erzeugtem Wasserstoff, Methan aus biologischer Quelle oder eine Mischung daraus.

Im Rahmen einer bevorzugten Ausführungsform des Verfahrens enthält der besagten Wasserstoff enthaltende Brennstoff bezogen auf sein Gesamtgewicht mehr als 25 Gew.-% mittels regenerativer Energie erzeugtes Wasserstoffgas, bevorzugt bezogen auf sein Gesamtgewicht mindestens 50 Gew.-% mittels regenerativer Energie erzeugtes Wasserstoffgas.

Im Rahmen einer bevorzugten Ausführungsform des Verfahrens enthält der Brennstoff bezogen auf sein Gesamtgewicht mehr als 25 Gew.-% Methan aus biologischer Quelle, bevorzugt bezogen auf sein Gesamtgewicht mindestens 50 Gew.-% Methan aus biologischer Quelle.

Besonders bevorzugte Ausführungsformen des Verfahrens sind vorzugsweise dadurch gekennzeichnet, dass mindestens 80 % der für das gesamte Verfahren eingesetzten Energie mittels aus regenerativer Energie erzeugten elektrischen Stroms durchgeführt wird, insbesondere elektrischen Stroms wahlweise erhalten durch Nutzung von Windkraft, Sonnenenergie oder Wasserkraft.

Wird eine Menge der benötigen Energie für den besagten Reformerprozess über eine elektrische Heizung eingebracht, so kann diese Energie über in den Reaktor eingebrachte Heizwendeln als Heizelement oder über die Reaktorwände als Heizelement erfolgen. In einer weiteren vorteilhaften Ausführung kann ein genutzter Katalysator für den besagten Reformerprozess auf den elektrisch beheizten Flächen aufgebracht sein.

Unter Berücksichtigung der erfindungsgemäßen Maßgabe kann die Beheizung des besagten Reformerprozesses unter Bereitstellung der erforderlichen Wärmeenergie über eine Verbrennung von Wasserstoff erfolgen, der in der H₂-CO Abtrenneinheit aus dem Kohlenmonoxid-haltigen Produktgas abgetrennt wird und/oder der mittels Wasserelektrolyse erzeugt wird.

Wenn Wärmeenergie für den besagten Reformerprozess durch Verbrennung von Wasserstoff-haltigem Brennstoff bereitgestellt wird, wird wiederum im Rahmen einer weiter bevorzugten Ausführungsform des Verfahrens der für die Verbrennung von Wasserstoff-haltigem Brennstoff eingesetzte Wasserstoff durch mindestens ein Elektrolyseverfahren bereitgestellt, ausgewählt aus der Wasserelektrolyse von Wasser zu Wasserstoff und Sauerstoff, der Chlor-Alkali-Elektrolyse, der Salzsäure-Elektrolyse, wobei die für das Elektrolyseverfahren genutzte elektrische Energie mittels regenerativer Energie erzeugt wird. Dabei ist es wiederum besonders bevorzugt, wenn als regenerative Energie wahlweise Windkraft, Sonnenenergie, Wasserkraft oder Mischungen daraus, eingesetzt wird. Die Bereitstellung von Wasserstoff erfolgt dabei aus einer Wasserelektrolyse oder aus einer Elektrolyse zur Herstellung von Chlor. Durch die Integration einer Wasserelektrolyse kann zusätzlich benötigter Wasserstoff zur Hydrierung der Nitro-Verbindungen zu Aminen hergestellt werden, die mit Phosgen zu Isocyanaten umgewandelt werden können.

Der bei der Wasserelektrolyse gebildete Sauerstoff kann zur Optimierung der Verbrennung des gewählten Brennstoffes im Rahmen der Bereitstellung der für den Reformerprozess notwendigen Wärmeenergie genutzt werden.

Die Wasserelektrolyse kann mit Anlagen nach dem Stand der Technik durchgeführt werden. Bekannt und kommerziell erhältlich sind technische Anlagen zur alkalischen Wasserelektrolyse als auch zur Polymerelektrolyt-basierten Elektrolyse, der so genannten PEM-Elektrolyse. Die Prinzipien der Wasserelektrolyse sind beispielhaft in Kapitel 6.3.4 in Volkmar M. Schmidt in "Elektrochemische Verfahrenstechnik" (2003 Wiley-VCH-Verlag; ISBN 3-527-29958-0) beschrieben.

Durch die eingesetzte Wärmeenergie wird die für den besagten Reformerprozess der Synthese von Kohlenmonoxid notwendige Temperatur von mindestens 500°C erreicht. Es hat sich herausgestellt, dass im Rahmen einer bevorzugten Ausführungsform eines effektiveren Verfahrens, die Synthese im Reformerprozess bei einer Temperatur von mindestens 700°C, besonders bevorzugt in einem Bereich von 800 bis 1100 °C, ganz besonders bevorzugt in einem Bereich von 850 bis 1000°C, abläuft.

Es hat sich als bevorzugt erwiesen, wenn in einer Ausführungsform des Verfahrens die Synthese im besagten Reformerprozess bei einem absoluten Druck in einem Bereich von 10 bis 40 bar, besonders bevorzugt von 15 bis 35 bar, ganz besonders bevorzugt 18 bis 25 bar, abläuft.

Zur Optimierung der Energetik sowie der Selektivität des besagten Reformerprozesses, findet im Rahmen einer bevorzugten Ausführungsform des Verfahrens die Synthese im besagten Reformerprozess unter Einsatz eines an einer festen Phase geträgerten Katalysators statt.

Bevorzugt für diese Ausführungsform verwendbare Katalysatoren enthalten Nickel. Weiter ist es möglich, dass der Katalysator neben Nickel weitere Metalle der d-Gruppen Elemente des Periodensystems enthält, insbesondere mindestens ein zusätzliches Metall, ausgewählt aus Cer, Mangan, Samarium, Lanthan oder Palladium.

Der Katalysator der bevorzugten Ausführungsform ist an einer festen Phase geträgert. Als feste Phase eignet sich beispielsweise mindestens ein Material ausgewählt aus der Gruppe, die gebildet wird aus anorganischen Materialien, bevorzugt aus feuerfesten Metalloxiden. Feuerfeste Oxide sind Metalloxide, die bei hohen Temperaturen von 500°C bis 1100°C stabil sind. Solche Oxide umfassen die Oxide von Aluminium, Silizium, Magnesium, Zirkonium, Titan, Chrom, Zink, Zinn und anderer Metalle oder Kombinationen von Aluminiumoxid mit Magnesiumoxid. Kristalline anorganische Materialien umfassen Aluminosilikate, Silicium-Aluminiumphosphate, Silicalit, Spinelle und andere natürliche Zeolithe und Tone.

Bevorzugt geeignete an einer festen Phase geträgerte Katalysatoren werden ausgewählt aus mindestens einem Vertreter der Gruppe, die gebildet wird aus Ni/gamma-Al₂O₃, Ni/Mn/gamma-Al₂O₃, Ni/CeO₂/YSZ, Ce_{0,7}La_{0,2}Ni_{0,1}O₂₋₆, Ni/Silicalite-1/SiC, Ni/Sm/SBA-15, Ni/CeO₂, Ni/MgAl₂O₄, Fe/Ni/Pd/MgAl₂O₄, Ni/Ce/MgAl₂O₄.

Der Katalysator kann durch diverse bekannte Methoden auf der festen Phase aufgebracht werden, beispielsweise durch Fällung, nasser Imprägnierung, mittels hydrothermal Verfahren oder Verbrennung.

Der Reformerprozess des erfindungsgemäßen Verfahrens kann in einem Schritt einer Reaktionsstufe ablaufen. In einer weiteren Ausführungsform des Verfahrens kann der Reformerprozess mindestens zwei Schritte umfassen
(a) Umsetzung von zumindest Methan und Wasserdampf zu einem Gemisch, mindestens enthaltend Kohlenmonoxid und Wasserstoff
(b) Umsetzung zumindest des besagten Gemisches aus (a) unter Zusatz von CO₂ zu Kohlenmonoxid-haltigem Produktgas.

Diese zwei Schritte können beispielsweise räumlich getrennt in zwei Reaktionsstufen durchgeführt werden, wobei zunächst in einer ersten Reaktionsstufe zumindest Methan und Wasserdampf zu einem Gemisch, mindestens enthaltend Kohlenmonoxid und Wasserstoff, umgesetzt werden und anschließend diesem Gemisch in einer zweiten Reaktionsstufe CO₂ zugesetzt und damit zu Kohlenmonoxid-haltigem Produktgas umgesetzt wird.

Das aus dem erfindungsgemäßen Verfahren erhältliche Kohlenmonoxid-haltige Produktgas des Reformprozesses enthält neben Kohlenmonoxid meistens Anteile von nicht umgesetztem CO₂, sowie von Wasser, Wasserstoffgas und Methan. Das Kohlenmonoxid-haltige Produktgas, wird mindestens durch Abtrennung von CO₂ und gegebenenfalls zusätzlich durch mindestens eine Abtrennung ausgewählt aus der Abtrennung von Wasser, der Abtrennung von Wasserstoff oder einer Kombination davon, gereinigt.

Das Kohlenmonoxid-haltige Produktgas wird folglich aufgereinigt und zu diesem Zweck zwingend einer Abtrennung von Kohlendioxid einer CO₂-Abtrenneinheit zugeführt, bei der das CO₂ abgetrennt und in den Reformerprozess zurückgeführt wird.

Die CO₂ Abtrenneinheit und damit die Abtrennung von CO₂, kann als "Aminwäsche" ausgeführt werden, wobei das Kohlenmonoxid-haltige Produktgas des Reformerprozesses hier insbesondere die grundsätzlich bekannte Wäsche des Gasgemisches nach dem Prinzip der Chemisorption mit Aminen, wie Monoethanolamin (MEA) Diethanolamin (DEA), Methyldiethanolamin (MDEA) oder Diglycolamin (DGA) durchläuft, welche in einer Absorptionskolonne eine hohe Reinheit des gereinigten Gasgemisches erreicht.

In einer erfindungsgemäß bevorzugten Ausführungsform des Verfahrens erfolgt eine Abtrennung von Wasser aus dem Kohlenmonoxid-haltigen Produktgas. Wenn in einer Ausführungsform des erfindungsgemäßen Verfahrens eine Wasserelektrolyse zur Bereitstellung von Wasserstoff und/oder Sauerstoff, beispielsweise für eine Brennkammer einer Vorrichtung zur Zuführung von Wärmeenergie des Reformers, genutzt wird, hat es sich als weiter bevorzugt erwiesen, das aus der besagten Abtrennung von Wasser abgetrennte Wasser in die Wasserelektrolyse zu rezyklieren.

Im Rahmen einer weiteren bevorzugten Ausführungsform des Verfahrens erfolgt die Abtrennung von Kohlendioxid nachdem zuvor Wasser aus dem Kohlenmonoxid-haltigen Produktgas abgetrennt wurde. Zu diesem Zweck wird das Kohlenmonoxid-haltige Produktgas des Reformerprozesses zunächst einer Wasser-Abtrenneinheit zugeführt, dort das Wasser abgetrennt und das nach der Abtrennung von Wasser erhaltene Kohlenmonoxid-haltige, trockene Produktgas einer CO₂-Abtrenneinheit zugeführt, bei der das CO₂ abgetrennt und in den Reformer zurückgeführt wird. In der Wasser-Abtrenneinheit wird zur Abtrennung des Wassers beispielsweise das dem Reformerprozess entnommene Kohlenmonoxid-haltige Produktgas abgekühlt und das Wasser, beispielsweise als Kondensat, abgetrennt.

Als erfindungsgemäß bevorzugte Variante des Verfahrens gilt ein Verfahren, in dem das (bevorzugt vorher von Wasser und von CO₂ befreite) Kohlenmonoxid-haltige Produktgas in eine H₂-CO Trenneinheit eingebracht wird, in der Wasserstoff abgetrennt wird. Dabei bildet sich zumindest ein Gasstrom, dessen Gas bei 25°C und 1013 mbar mindestens 95 Vol.% Kohlenmonoxid, bevorzugter mindestens 99 Vol.% Kohlenmonoxid, enthält. Das besagte eingebrachte Produktgas wird in der H₂-CO Trenneinheit vorzugsweise zunächst in zwei Gasströme aufgetrennt. Es entsteht dabei ein Gas in Form eines Gasstroms, das mindestens 95 Vol.% (bevorzugt mindestens 99 Gew.-%) Kohlenmonoxid enthält, ein weiteres Gas in Form eines Gasstroms, dessen größter Bestandteil Wasserstoff ist und unter anderem Kohlenmonoxid und Methan enthält. Das weitere Gas wird auch als Restgas der H₂-CO Trennung oder falls keine Restgasbehandlung erfolgt, als Endgas bezeichnet. Eine nach diesem Prinzip der Auftrennung arbeitende H₂-CO Trenneinheit ist die sogenannte Coldbox. Das Wasserstoff-haltige Restgas der H₂-CO Trennung kann zur Aufkonzentrierung des Wasserstoffs einer anschließenden Restgasbehandlung ausgesetzt werden. Nach der Aufarbeitung durch die Restgasbehandlung wird ein mit Wasserstoffgas angereichertes Gas in Form eines Gasstroms und ein weiteres Gas - das sogenannte Restgas der Restgasbehandlung oder Endgas - in Form eines Gasstroms, erhalten, das eine Mischung von CO, Methan und einer geringeren Menge Wasserstoff enthält, als das mit Wasserstoffgas angereicherte Gas.

Das Endgas kann als Restgas der H₂-CO Trennung oder Restgas der Aufarbeitung im Rahmen einer weiteren Ausführungsform als Brennstoff zur Bereitstellung von Wärmeenergie des Reformerprozesses in der Brennkammer des Heizelementes oder zur Wasserdampf-Erzeugung genutzt werden.

Soll das aus dem Kohlenmonoxid-haltigen Produktgas erhaltene Kohlenmonoxid einen hohen Reinheitsgrad besitzen und bevorzugt in einer anschließenden Phosgensynthese eingesetzt werden, hat es sich im Rahmen einer besonders bevorzugten Ausführungsform als geeignet erwiesen, die Aufreinigung des Kohlenmonoxid-haltigen Produktgases des Reformerprozesses durch Abtrennung von Wasser, Abtrennung von CO₂ und Abtrennung von Wasserstoff in der folgenden Reihenfolge auszuführen,
a1) Abtrennung von Wasser aus dem Kohlenmonoxid-haltigen Produktgas des Reformerprozesses unter Erhalt eines getrockneten, Kohlenmonoxid-haltigen Produktgases,
a2) danach Abtrennung von CO₂ aus dem getrockneten, Kohlenmonoxid-haltigen Produktgas, unter Erhalt eines vorgereinigten Kohlenmonoxid-haltigen Produktgases,
a3) danach Abtrennung von Wasserstoff aus dem vorgereinigten Kohlenmonoxid-haltigen Produktgas unter Erhalt eines Kohlenmonoxid-haltigen Produktgases, enthaltend bei 25°C und 1013 mbar mindestens 95 Vol.% Kohlenmonoxid.

Das aus der Abtrennung von Wasserstoff abgetrennte Wasserstoffgas des erfindungsgemäßen Verfahrens kann für die Hydrierung von Nitroverbindungen zur Herstellung von Aminen als Rohstoff für die Isocyanatsynthese eingesetzt werden. Im Rahmen einer bevorzugten Ausführungsform des Verfahrens wird nach der Abtrennung von Wasserstoff in der H₂/CO-Trennung, insbesondere unter Verwendung einer Coldbox, der abgetrennte gasförmige Wasserstoff aus dem Restgas der H₂-CO Trennung nach weiterer Restgasbehandlung als daraus hervorgehender Wasserstoff in eine Hydrierung von organischer Nitroverbindung zur Herstellung von organischem Amin als Rohstoff für organisches Isocyanat eingespeist.

Wie erwähnt erfolgt im Rahmen einer bevorzugten Ausführungsform des Verfahrens zusätzlich eine Synthese von Phosgen durch Umsetzung von zumindest dem zuvor nach der Reinigung bereitgestellten Kohlenmonoxid und Chlor. Insbesondere wird dabei bevorzugt die besagte Reinigung gemäß für einen hohen Reinheitsgrad geeigneten Ausführungsform durchgeführt und das verbliebene Kohlenmonoxid aus der Reinigung in die Phosgensynthese eingespeist.

Für die Bereitstellung des Chlors für die im Rahmen dieser Ausführungsform erfolgende Synthese von Phosgen ist dem Fachmann die Herstellung von Chlorgas aus elektrochemischer Oxidation nach der Salzsäure-Elektrolyse mit Gasdiffusionselektrode (auch als HCl ODC Elektrolyse-Verfahren bezeichnet (ODC steht für Oxygen Depleting Electrode, als eine ODC wird beispielsweise die sogenannte SVK (Sauerstoffverzehrkathode) eingesetzt); geeignete Elektrolysezelle vgl. US,6022,634 A, WO 03/31690 A1), die Herstellung von Chlorgas aus Salzsäure-Diaphragmaelektrolyse (vgl. EP 1 103 636 A1), die Herstellung von Chlorgas aus thermokatalytischer Gasphasenoxidation (vgl. WO 2012/025483 A2), sowie die Herstellung von Chlor aus Chloralkali-Elektrolyse (vgl. WO 2009/007366 A2) hinlänglich bekannt. Auf den Inhalt der vorgenannten, im Zusammenhang mit der Herstellung von Chlorgas zitierten Schriften wird ausdrücklich und vollumfänglich Bezug genommen. Das für die Synthese von Phosgen benötigte Chlor wird in einer bevorzugten Variante dieser Ausführungsform des Verfahrens elektrolytisch hergestellt, insbesondere durch elektrochemische Oxidation nach der Salzsäure-Elektrolyse mit Gasdiffusionselektrode, durch elektrochemische Oxidation nach der Salzsäure-Diaphragmaelektrolyse oder durch elektrochemische Oxidation nach der Chloralkali-Elektrolyse. Dabei ist es wiederum besonders bevorzugt, wenn die besagte elektrochemische Oxidation jeweils unter Verwendung von aus regenerativer Energie erzeugtem elektrischem Strom durchgeführt wird, insbesondere aus regenerativer Energie in Form von Windkraft, Sonnenenergie oder Wasserkraft.

Das durch die Synthese von Phosgen gebildete Phosgen wird im Rahmen einer besonders bevorzugten Ausführungsform des Verfahrens in einem weiteren Schritt zu einem organischen Isocyanat umgesetzt. Daher erfolgt im Rahmen dieser Ausführungsform eine Umsetzung von aus besagter Synthese von Phosgen stammendem Phosgen mit mindestens einem organischen Amin zu mindestens einer organischen Isocyanat-Verbindung und Chlorwasserstoff.

Es sind generell solche erfindungsgemäßen Verfahren dieser Ausführungsform bevorzugt, in denen das gewonnene organische Isocyanat mindestens zwei Isocyanatgruppen enthält. Zu diesem Zweck wird bei der Synthese wiederum bevorzugt als Reaktand organisches Amin mit mindestens zwei Aminogruppen eingesetzt.

Besonders bevorzugt enthält das gewonnene organische Isocyanat mindestens zwei Isocyanatgruppen und weist eine Molmasse von höchstens 1000 g/mol, insbesondere von höchstens 800 g/mol, auf.

Ganz besonders bevorzugt eingesetzte organische Amine werden ausgewählt aus Toluylendiamin (TDA), Methylendi(phenylamin) (MDA) (wiederum bevorzugt ausgewählt aus Diphenylmethan-2,2'-diamin, Diphenylmethan-2,4'-diamin, Diphenylmethan-4,4'-diamin oder Mischungen daraus), Hexamethylendiamin, Isophorondiamin, 1,3-Bis(aminomethyl)benzol, Cyclohexyldiamin oder Mischungen daraus, wobei TDA, MDA oder Mischungen daraus ganz besonders bevorzugte organische Isocyanate sind.

Ganz besonders bevorzugt erhaltene organische Isocyanate werden ausgewählt aus Toluylendiisocyanat (TDI), Methylendi(phenylisocyanat) (MDI) (wiederum bevorzugt ausgewählt aus Diphenylmethan-2,2'-diisocyanat, Diphenylmethan-2,4'-diisocyanat, Diphenylmethan-4,4'-diisocyanat oder Mischungen daraus), Hexamethylendiisocyanat (HDI), Isophorondiisocyanat (IPDI), 1,3-Bis(isocyanatomethyl)benzol (XDI), Cyclohexyldiisocyanat (CHDI) oder Mischungen daraus, wobei TDI, MDI oder Mischungen daraus ganz besonders bevorzugte organische Isocyanate sind.

Ferner entsteht im Rahmen der Ausführungsform mit Synthese von organischem Isocyanat neben besagtem organischen Isocyanat zusätzlich Chlorwasserstoff. Es ist wiederum bevorzugt diesen Chlorwasserstoff als Edukt der Herstellung von Chlor für die besagte Synthese von Phosgen zuzuführen. Die dafür notwendige Abtrennung und Reinigung des in der Isocyanat-Herstellung gebildeten Chlorwasserstoffs kann anschließend erfolgen durch eine oxidative Umsetzung in einer thermokatalytischen Gasphasenoxidation mit Sauerstoff zu Chlor und Wasser und ggf. Nutzung von O₂ aus der Wasserelektrolyse.

Eine weitere alternative Vorgehensweise bei der Nutzbarmachung des Chlorwasserstoffs als Edukt für die Herstellung von Chlor ist die Umsetzung des Chlorwasserstoffes mit Wasser zu Salzsäure und anschließender elektrochemischer Oxidation der Salzsäure zu Chlor und ggf. Wasserstoff. Diese wässrige Salzsäure wird insbesondere der zuvor beschriebenen elektrochemischen Oxidation nach der Salzsäure-Elektrolyse mit Gasdiffusionselektrode, der elektrochemischen Oxidation nach der Salzsäure-Diaphragmaelektrolyse oder der elektrochemischen Oxidation nach der Chloralkali-Elektrolyse) zugeführt. Im Falle der Salzsäure-Elektrolyse mit Gasdiffusionselektrode benötigte O₂ kann aus der Wasserelektrolyse bezogen werden.

Der anfallende Chlorwasserstoff aus der Isocyanat-Herstellung kann ebenso nach Reinigung und Absorption in Wasser zu Salzsäure umgesetzt und die resultierende Salzsäure in den Markt für diverse Anwendungen verkauft werden.

Im Rahmen einer besonders bevorzugten Ausführungsform des Verfahrens wird zusätzlich
eine Synthese von Phosgen durch Umsetzung von zumindest dem zuvor nach der Reinigung des besagten Kohlenmonoxid-haltigen Produktgases bereitgestellten Kohlenmonoxid und Chlor;
eine Isocyanat-Herstellung durch Umsetzung des erhaltenen Phosgens mit mindestens einem organischen Amin zu mindestens einer organischen Isocyanat-Verbindung und Chlorwasserstoff;
eine Abtrennung und Reinigung des in der Isocyanat-Herstellung gebildeten Chlorwasserstoffs und eine anschließende oxidative Umsetzung des Chlorwasserstoffs, ausgewählt aus
   - einer Umsetzung von Chlorwasserstoff in einer thermokatalytischen Gasphasenoxidation mit Sauerstoff zu Chlor und Wasser gegebenenfalls unter Nutzung von Sauerstoff aus der Wasserelektrolyse,
   - einer Umsetzung von Chlorwasserstoff mit Wasser zu Salzsäure und anschließender elektrochemischer Oxidation der Salzsäure zu Chlor und gegebenenfalls Wasserstoff;
eine an die oxidative Umsetzung des Chlorwasserstoffs anschließende Zuführung des gebildeten Chlors in die besagte Synthese von Phosgen, gegebenenfalls unter Zuführung einer zusätzlichen Menge Chlor in die besagte Synthese von Phosgen, die aus einer Chloralkali-Elektrolyse stammt;
durchgeführt.

Für alle Ausführungsformen des Verfahrens, die eine Synthese von Phosgen als Schritt enthaltenen, ist es weiter bevorzugt eine elektrochemische Oxidation (Elektrolyse) für die Chlorherstellung unter Verwendung von aus regenerativer Energie erzeugtem elektrischen Strom durchzuführen, insbesondere von elektrischem Strom wahlweise erhalten durch Nutzung von Windkraft, Sonnenenergie oder Wasserkraft.

In einer anderen bevorzugten Ausführungsform des neuen Verfahrens werden mindestens eine Wasserelektrolyse und/oder mindestens eine elektrochemische Oxidation (Elektrolyse) für die Chlorherstellung unter Verwendung von elektrischem Strom aus rückgekoppelter Energie durchgeführt, die mit der Nutzung des Wasserdampfes aus dem Reformerprozess erhalten wird.

Die in der Ausführungsform des Verfahrens für die Isocyanatherstellung eingesetzten organischen Amine werden wiederum bevorzugt durch einen zusätzlichen Schritt der Hydrierung von organischen Nitroverbindungen mit gasförmigem Wasserstoff bereitgestellt. Dabei eingesetzter Wasserstoff stammt bevorzugt aus einer Wasserelektrolyse und/oder aus der Reinigung des Kohlenmonoxid-haltigen Produktgases als abgetrennter Wasserstoff nach dem Schritt der Abtrennung von Wasserstoff.

In einer besonders bevorzugten Ausführungsform des erfindungsgemäßen Verfahrens wird Wasserstoff aus einer Wasserelektrolyse in die Hydrierung von organischer Nitroverbindung für die Herstellung von organischem Amin eingespeist. Besonders bevorzugt wird dabei der Wasserstoff durch eine Wasserelektrolyse unter Verwendung von elektrischem Strom aus regenerativer Energie (insbesondere aus regenerativer Energie in Form von Windkraft, Sonnenenergie oder Wasserkraft) gebildet und einem Schritt zur Hydrierung von mindestens einer organischen Nitroverbindung zugeführt, wobei das bei der Hydrierung von besagter Nitroverbindung erhaltene mindestens eine organische Amin mit Phosgen unter Erhalt mindestens eines organischen Isocyanates umgesetzt wird.

Es ist erfindungsgemäß besonders bevorzugt, zumindest den bei der Reinigung aus dem Kohlenmonoxid-haltigen Produktgas abgetrennten Wasserstoff einem Hydrierungsschritt von mindestens einer organischen Nitroverbindung zuzuführen. Hierbei wird ebenso mindestens ein organisches Amin für die Herstellung von organischem Isocyanat zugänglich.

Die erhaltenen organischen Isocyanate können in einem zusätzlichen Schritt des Verfahrens mit mindestens einem Polyol, insbesondere mindestens einem Polyesterpolyol oder Polyetherpolyol, zu Polyurethan-Materialien umgesetzt werden.

Entsprechend hergestellte Polyurethan-Materialien sind beispielsweise in Form von Schäumen, Lacken, Isoliermasse, Bestandteil von Marktprodukten.

Durch das erfindungsgemäße Verfahren kann der Einsatz von fossilen Rohstoffen für die Synthese von Kohlenmonoxid und im Rahmen weiterer Ausführungsformen für die Isocyanat-Herstellung reduziert werden und es kann in verbessert nachhaltiger Weise Kohlenmonoxid bzw. Isocyanat und wiederum daraus Polyurethan-Material und damit gefertigte Produkte hergestellt werden.

Das erfindungsgemäße Verfahren kann bevorzugt mit einer erfindungsgemäßen Vorrichtung als weiterem Gegenstand der Erfindung ausgeführt werden, wobei diese Vorrichtung für die Synthese von Kohlenmonoxid in einem Reformerprozess (6) ausgestaltet ist, worin Methan mit Wasserdampf unter Zusatz von mindestens CO₂, unter Zuführung von Wärmeenergie bei einer Temperatur von mindestens 500°C zu Kohlenmonoxid-haltigem Produktgas umgesetzt wird, und
die für die Reinigung des aus vorgenannter Synthese erhaltenen Kohlenmonoxid-haltigen Produktgases ausgestaltet ist, mindestens durch Abtrennung von CO₂ und gegebenenfalls zusätzlich durch mindestens eine Abtrennung ausgewählt aus der Abtrennung von Wasser, der Abtrennung von Wasserstoff oder einer Kombination davon, unter Erhalt von Kohlenmonoxid,
zur Bereitstellung von Kohlenmonoxid für die Herstellung von organischem Isocyanat, nach einem Verfahren, umfassend zumindest die Schritte
Synthese von Phosgen aus zumindest Kohlenmonoxid und Chlor,
Umsetzung von Phosgen mit mindestens einem organischen Amin zu mindestens einer organischen Isocyanat-Verbindung und Chlorwasserstoff.

Eine bevorzugte Ausführungsform für eine entsprechend geeignete Vorrichtung für die Synthese von Kohlenmonoxid unter Bereitstellung von Kohlenmonoxid für die Herstellung von Phosgen für die Synthese von organischem Isocyanat umfasst mindestens folgende Vorrichtungsteile
- mindestens einen Reformer, enthaltend mindestens einen Reaktionsraum, mindestens ein Heizelement als eine Vorrichtung zur Zuführung von Wärmeenergie zum Reaktionsraum, sowie mindestens einen Einlass für die Einbringung von Methan, Wasserdampf und CO₂ in den Reaktionsraum und mindestens einen Auslass für das Kohlenmonoxid-haltige Produktgas aus dem Reformer;
- mindestens eine Vorrichtung zur Abtrennung von Wasser, die zumindest einen Einlass aufweist, der in Fluidverbindung mit dem Auslass für das Kohlenmonoxid-haltige Produktgas aus dem Reformer steht und mindestens einen Auslass für das abgetrennte Wasser und mindestens einen Auslass für das getrocknete, Kohlenmonoxid-haltigen Produktgas;
- mindestens eine Vorrichtung zur Abtrennung von CO₂, die zumindest einen Einlass aufweist, der in Fluidverbindung mit dem Auslass für das getrocknete, Kohlenmonoxid-haltige Produktgas aus der Vorrichtung zur Abtrennung von Wasser steht und mindestens einen Auslass für das abgetrennte CO₂, der in Fluidverbindung mit einem Einlass für CO₂ des Reformers steht und mindestens einen Auslass für das vorgereinigte, Kohlenmonoxid-haltige Produktgas;

- mindestens eine Vorrichtung zur Trennung von H₂-CO, die zumindest einen Einlass aufweist, der in Fluidverbindung mit dem Auslass für das vorgereinigte, Kohlenmonoxid-haltige Produktgas aus der Vorrichtung zur Abtrennung von CO₂ steht und mindestens einen Auslass für das Kohlenmonoxid und mindestens einen Auslass für das Restgas der H₂-CO Trennung;
- mindestens eine Vorrichtung zur Herstellung von Phosgen, die mindestens einen Einlass für Kohlenmonoxid aufweist, der in Fluidverbindung mit dem für das Kohlenmonoxid vorgesehenen Auslass der Vorrichtung zur Trennung von H₂-CO in Fluidverbindung steht, sowie mindestens einen Einlass für Chlor und mindestens einen Auslass für Phosgen enthält;
- mindestens eine Vorrichtung zur Herstellung von Chlor, die mindestens einen Einlass für Chlorwasserstoff aufweist, sowie mindestens einen Auslass für Wasserstoff und mindestens einen Auslass für Chlor enthält, der mit einem Einlass für Chlor von der Vorrichtung zur Herstellung von Phosgen in Fluidverbindung steht;
mit der Maßgabe, dass
i) das Heizelement des Reformers in Fluidverbindung mit mindestens einer Quelle, ausgewählt aus einer Quelle für aus regenerativer Energie bereitgestelltem Wasserstoff als Brennstoff, einer Quelle für aus biologischer Quelle bereitgestelltem Methan als Brennstoff, steht oder das Heizelement an eine Quelle zur Bereitstellung von elektrischer Energie aus regenerativer Energie für die Umwandlung in Wärmeenergie angeschlossen ist; und/oder
ii) ein Einlass des Reaktors in Fluidverbindung mit einer weiteren CO₂-Quelle zur Einbringung von zusätzlichem CO₂ in den Reaktionsraum steht, herangezogen wird.

Unter einer "Fluidverbindung" wird erfindungsgemäß ein Vorrichtungsteil verstanden, der Anlagenteile miteinander verbindet und durch die sich ein Stoff, der in jedem Aggregatzustand vorliegen kann, von einem Anlagenteil zu einem anderen Anlagenteil durch einen Stoffstrom transportieren lässt, beispielsweise eine Zuleitung in Form eines Rohres, die durch weitere Anlagenteile unterbrochen sein kann.

Es ist analog zum zuvor beschriebenen Verfahren im Rahmen einer bevorzugten Ausführungsform der Vorrichtung ebenfalls bevorzugt, wenn der Auslass für das Restgas der Vorrichtung zur H₂-CO Trennung mit dem Heizelement des Reformers, insbesondere der Brennkammer des Heizelementes, in Fluidverbindung steht. Für eine Ausnutzung des Wasserstoffgases aus dem Restgas der Vorrichtung der H₂-CO Trennung für Synthesezwecke ist es bevorzugt, wenn der Auslass für das Restgas der Vorrichtung der H₂-CO Trennung mit dem Einlass einer Vorrichtung zur Restgasbehandlung in Fluidverbindung steht, wobei besagte Vorrichtung zur Restgasbehandlung mindestens einen Auslass für Wasserstoffgas und mindestens einen Auslass für Restgas der Restgasbehandlung besitzt. In diesem Falle ist es bevorzugt, wenn der Auslass für das Restgas der Vorrichtung für die Restgasbehandlung in Fluidverbindung mit dem Heizelement des Reformers, insbesondere mit der Brennkammer des Heizelementes, steht.

Für die Synthese von organischem Isocyanat ist es im Rahmen einer bevorzugten Ausführungsform der Vorrichtung vorteilhaft, wenn der Auslass für Phosgen der Vorrichtung zur Herstellung von Phosgen mit einer Vorrichtung zur Herstellung von organischem Isocyanat in Fluidverbindung steht, wobei die Vorrichtung zur Herstellung von organischem Isocyanat neben mindestens einem Einlass für Phosgen zusätzlich mindestens einen Einlass für organisches Amin und mindestens einen Auslass für organisches Isocyanat besitzt. Dabei ist es besonders bevorzugt, wenn die Vorrichtung zur Herstellung von organischem Isocyanat über mindestens einen Einlass für organisches Amin mit einer Vorrichtung zur Hydrierung von organischer Nitroverbindung in Fluidverbindung steht, wobei die Vorrichtung zur Hydrierung organischer Nitroverbindung mindestens einen Einlass für Wasserstoffgas, mindestens einen Einlass für organische Nitroverbindung und mindestens einen Auslass für organisches Amin aufweist. Als Quelle für das Wasserstoffgas zur Hydrierung kann vorteilhafter Weise das Wasserstoffgas aus der Restgasbehandlung des Restgases der Vorrichtung der H₂-CO Trennung dienen. Zu diesem Zweck stehen bevorzugt der Auslass für Wasserstoffgas der Vorrichtung zur Restgasbehandlung und der Einlass für Wasserstoffgas der Vorrichtung zur Hydrierung von organischer Nitroverbindung in Fluidverbindung. Zusätzlich oder in Ergänzung dazu dient besonders bevorzugt als Quelle für das Wasserstoffgas zur Hydrierung das Wasserstoffgas aus der Vorrichtung zur Herstellung von Chlor. Zu diesem Zweck stehen besonders bevorzugt der Auslass für Wasserstoffgas der Vorrichtung zur Herstellung von Chlor und der Einlass für Wasserstoffgas der Vorrichtung zur Hydrierung von organischer Nitroverbindung in Fluidverbindung.

Es ist analog zum zuvor beschriebenen Verfahren im Rahmen einer bevorzugten Ausführungsform der Vorrichtung ebenfalls bevorzugt, wenn die erfindungsgemäße Vorrichtung zusätzlich eine Vorrichtung zur Wasserelektrolyse enthält, die neben mindestens einer Anode und mindestens einer Kathode, zusätzlich mindestens einen Einlass für Wasser, mindestens einen Auslass für Wasserstoffgas, mindestens einen Auslass für Sauerstoffgas aufweist und an eine Quelle für elektrischen Strom aus regenerativer Energie angeschlossen ist, und deren Auslass für Wasserstoffgas in Fluidverbindung mit einem Einlass für Wasserstoffgas einer Vorrichtung zur Hydrierung von organischer Nitroverbindung steht.

Ein weiterer Gegenstand der Erfindung ist die Verwendung einer Vorrichtung, die für die Synthese von Kohlenmonoxid in einem Reformerprozess ausgestaltet ist, worin Methan mit Wasserdampf unter Zusatz von mindestens CO₂, unter Zuführung von Wärmeenergie bei einer Temperatur von mindestens 500°C zu Kohlenmonoxid-haltigem Produktgas umgesetzt wird, und die für die Reinigung des aus vorgenannter Synthese erhaltenen Kohlenmonoxid-haltigen Produktgases ausgestaltet ist, mindestens durch Abtrennung von Wasser, durch Abtrennung von CO₂, durch Abtrennung von Wasserstoff oder durch eine Kombination von mindestens zwei der vorgenannten Abtrennungen in jeweils eine entsprechenden Abtrenneinheit, unter Erhalt von Kohlenmonoxid,
zur Bereitstellung von Kohlenmonoxid für die Herstellung von organischem Isocyanat, nach einem Verfahren, umfassend zumindest die Schritte
Synthese von Phosgen aus zumindest Kohlenmonoxid und Chlor,
Umsetzung von Phosgen mit mindestens einem organischen Amin zu mindestens einer organischen Isocyanat-Verbindung und Chlorwasserstoff.

Dabei ist es bevorzugt, die zuvor genannten bevorzugten weiteren Ausführungsformen von Merkmalen der Vorrichtung einzeln oder in Kombination als bevorzugte Ausführungsformen der Verwendung zu nutzen.

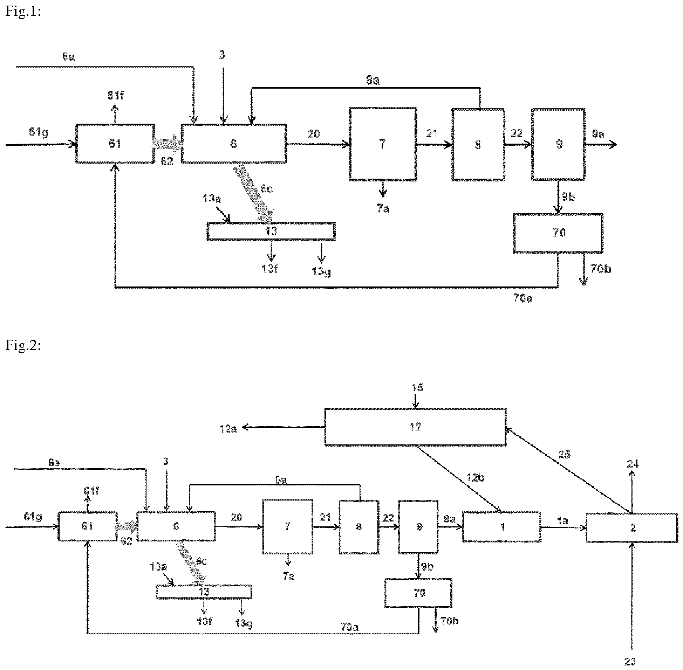

Die Erfindung wird nachstehend anhand der Figuren 1 bis 12 beispielhaft näher erläutert. In den Figuren haben die folgenden Bezugszeichen die jeweils rechtsstehende Bedeutung:
1 Phosgensynthese
1a Phosgen
2 Isocyanatherstellung
3 Wasserdampf für den Reformprozess 6
4 CO₂ Gasreinigung
4a CO₂ aus der CO₂ Gasreinigung 4
5 Wasserelektrolyse
5a Wasserstoff aus Wasserelektrolyse 5
5b Sauerstoff aus der Wasserelektrolyse 5
5c Wasser für die Wasserelektrolyse 5
6 Reformerprozess zur Synthese von Kohlenmonoxid
6a Methan zum Reformerprozess 6 für chemische Umsetzung
6c Wärme aus dem Reformerprozess 6 zur Wasserdampferzeugung 13
7 Wasser-Abtrenneinheit / Abtrennung von Wasser
7a Abgetrenntes Wasser aus der Wasser-Abtrenneinheit
8 CO₂ Abtrenneinheit / Abtrennung von CO₂
8a CO₂ aus der Abtrennung von CO₂
9 H₂-CO Trenneinheit / H₂-CO Trennung
9a Kohlenmonoxid CO aus der H₂-CO Trennung 9
9b Restgas der H₂-CO Trennung 9 (H₂, CO, CH₄ etc.)
9c Restgas der H₂-CO Trennung 9 zur Dampferzeugung
9d Restgas der H₂-CO Trennung 9 zur Brennkammer
10 Wasserstoff für die Hydrierung von Nitroaromaten 32
11 Wasserstoff für weitere Nutzung
12 Chlor-Herstellung
12a H₂ aus der Chlor-Herstellung
12b Cl₂ aus der Chlor-Herstellung
13 Energie- und Dampferzeugung
13a Erdgas für Dampferzeugung
13f CO₂ aus Dampferzeugung
13g Dampf
14 mittels regenerativer Energie hergestellte elektrische Energie für Wasserelektrolyse 5
15 mittels regenerativer Energie hergestellte elektrische Energie für die elektrolytische Herstellung von Chlor
20 Kohlenmonoxid-haltiges Produktgas aus dem Reformerprozess 6 (enthaltend H₂O, CO, H₂, CO₂, Nebenprodukte)
21 Kohlenmonoxid-haltiges Produktgas aus der Abtrennung von Wasser 7
22 vorgereinigtes Kohlenmonoxid-haltiges Produktgas aus der Abtrennung von CO₂ 8
23 organisches Amin
24 organisches Isocyanat
25 Chlorwasserstoff
32 Hydrierung organischer Nitroverbindung60 CO₂ aus weiteren Quellen
61 Heizelement und Wärmeenergiebereitstellung für den Reformerprozess 6, beispielsweise Brennkammer für Verbrennung von Brennstoff und/oder elektrisches Heizelement
61a Erdgas zur Brennkammer
61b Methan aus biologischer Quelle (Bio-Methan) zur Brennkammer 61c mittels regenerativer Energie hergestellte elektrische Energie zum Heizen des Heizelementes 61
61d mittels regenerativer Energie hergestelltes H₂ zur Brennkammer des Heizelementes 61
61f Abgas aus der Brennkammer des Heizelementes 61 (z.B. CO₂, H₂O)
61g Energie für Betreibung des Heizelementes mindestens ausgewählt aus 61b, 61c, 61d oder Mischungen daraus
62 Wärme erzeugt im Heizelement 61 für den Reformerprozess 6
70 Restgasbehandlung
70a Restgas (Endgas) der Restgasbehandlung 70 zur Brennkammer
70b Wasserstoff aus der Restgasbehandlung 70
70c Restgas der Restgasbehandlung 70 zur Dampferzeugung 13
70e Wasserstoff aus der Restgasbehandlung 70 für die Hydrierung von Nitroaromaten 32

Pfeile in den Figuren symbolisieren den Fluss von Stoffen, Energie oder Wärme zwischen Verfahrensschritten bzw. Vorrichtungsteilen, in denen die entsprechenden Verfahrensschritte ablaufen. Fig.1 zeigt eine Ausführungsform des Verfahrens für die Herstellung von Kohlenmonoxid aus Methan (6a), Wasserdampf und CO₂ (8a) für die Darstellung von Phosgen zur Synthese von organischem Isocyanat, enthaltend zumindest die Schritte
Synthese von Kohlenmonoxid in einem Reformerprozess (6), worin Methan und Wasserdampf (3) unter Zusatz von mindestens CO₂ (8a),
   unter Zuführung von Wärmeenergie (62) bei einer Temperatur von mindestens 500°C zu Kohlenmonoxid-haltigem Produktgas (20) umgesetzt wird,
Reinigung des aus vorgenannter Synthese erhaltenen Kohlenmonoxid-haltigen Produktgases (20), durch
   a1) Abtrennung von Wasser (7) aus dem Kohlenmonoxid-haltigen Produktgas (20) des Reformerprozesses (6) unter Erhalt eines getrockneten, Kohlenmonoxid-haltigen Produktgases (21), und Abführung des abgetrennten Wassers (7a);
   a2) danach Abtrennung von CO₂ (8) aus dem getrockneten, Kohlenmonoxid-haltigen Produktgas (21), unter Erhalt eines vorgereinigten Kohlenmonoxid-haltigen Produktgases (22), und Rückführung des abgetrennten CO₂ (8a) in den vorgenannten Reformerprozess (6);
   a3) danach Abtrennung von Wasserstoff (9) aus dem vorgereinigten Kohlenmonoxid-haltigen Produktgas (22) unter Erhalt von Kohlenmonoxid (9a);
mit der Maßgabe, dass die der Synthese von Kohlenmonoxid (9a) dem Reformerprozess (6) zugeführte Wärmeenergie (62) durch mindestens eine Methode (61g) bereitgestellt wird, ausgewählt aus (i) der Verbrennung von Brennstoff, der mittels regenerativer Energie erzeugten Wasserstoff enthält, (ii) der Verbrennung von Brennstoff, der Methan aus biologischer Quelle enthält, (iii) Umwandlung von aus regenerativer Energie erzeugter, elektrischer Energie (6c) in Wärme.

Aus der Abtrennung von Wasserstoff (9) wird gemäß Fig.1 das Wasserstoff-haltige Restgas (9b) durch eine Restgasbehandlung (70) gereinigt und der erhaltene Wasserstoff (70a) als Brennstoff in die Brennkammer des Heizelementes (61) geführt. Es ist ebenso denkbar, das Restgas (9b) direkt in die Brennkammer des Heizelementes zu führen (nicht gezeigt). Das von der Brennkammer emittierte CO₂ (61f) wird aus der Brennkammer herausgeführt. Als Quelle für das Methan (6a) wurde Erdgas gewählt, es ist jedoch bevorzugt, Methan aus biologischer Quelle zu wählen (61b). Der dem Reformerprozess zugesetzte Wasserdampf (3) wurde in der Dampferzeugung (13) unter Einsatz von Restwärme aus dem Reformerprozess und von Methan als Brennstoff (13a) bereitgestellt.

Fig.2 zeigt eine Ausführungsform eines in Fig. 1 dargestellten Verfahrens, welches in eine Herstellung von organischem Isocyanat (24) und den dafür notwendigen Rohstoff Phosgen (1a) eingebettet ist. Zu diesem Zweck wird das nach dem in Fig.1 illustrierten Verfahren hergestellte Kohlenmonoxid (9a) in eine Phosgenherstellung (1) eingebracht und dort gemeinsam mit dem in die Phosgenherstellung (1) eingebrachten Chlor (12b), das in einer unter Einbringung von aus regenerativer Energie produzierter elektrischer Energie (15) betriebenen Chlorherstellung (12) elektrolytisch aus Chlorwasserstoff gewonnen wird, zu Phosgen (1a) umgesetzt, wobei in einem anschließenden Schritt in der Isocyanatherstellung (2) das Phosgen (1a) gemeinsam mit einem organischen Amin (23) zum organischen Isocyanat (24) umgesetzt wird. Der in der Isocyanatherstellung (2) freigesetzte Chlorwasserstoff (25) wird der Chlorherstellung (12) zugeführt und der bei der Chlorherstellung anfallende Wasserstoff (12a) wird abgeführt. Der Wasserstoff (12a) kann beispielsweise als Brennstoff (61g) für das Heizelement (61) des Reformerprozesses (6) dienen (nicht in Fig.2 dargestellt).

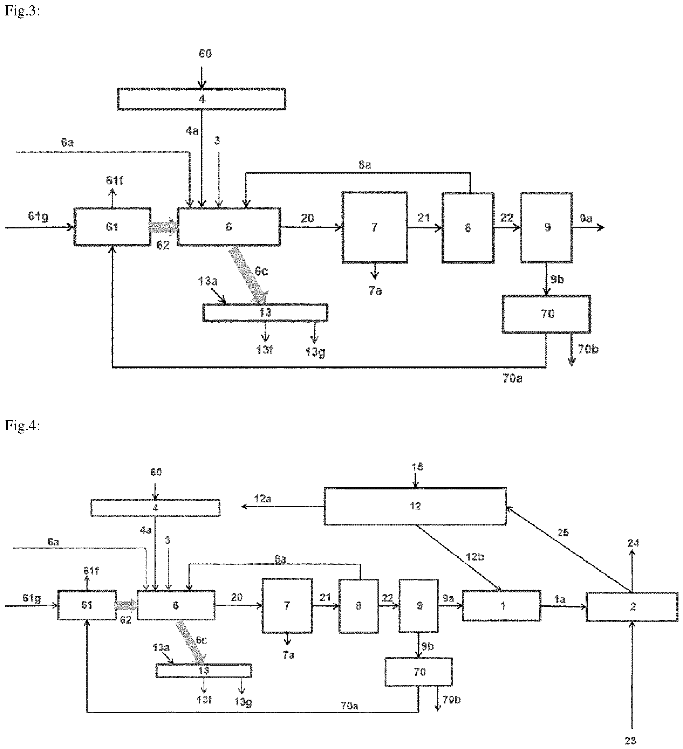

In Fig.3 wird eine Ausführungsform des in Fig.1 dargestellten Verfahrens gezeigt, in dem zusätzlich zum dem Reformerprozess (6) zugesetzten CO₂ (8a) weiteres CO₂ (4a) aus einer weiteren CO₂-Quelle (60) zugesetzt wird, wobei dieses CO₂ zuvor in einer CO₂ Gasreinigung (4) gereinigt wird. Spendet die weitere CO₂-Quelle (60) bereits gereinigtes CO₂, kann die CO₂ Gasreinigung (4) entfallen.

Fig.4 zeigt eine Ausführungsform eines in Fig. 2 dargestellten Verfahrens, welches in eine Herstellung von organischem Isocyanat (24) und den dafür notwendigen Rohstoff Phosgen (1a) eingebettet ist. Zu diesem Zweck wird das nach dem in Fig.2 illustrierten Verfahren hergestellte Kohlenmonoxid (9a) in eine Phosgenherstellung (1) eingebracht und dort gemeinsam mit dem in die Phosgenherstellung (1) eingebrachten Chlor (12b), das in einer unter Einbringung von aus regenerativer Energie produzierter elektrischer Energie (15) betriebenen Chlorherstellung (12) elektrolytisch aus Chlorwasserstoff gewonnen wird, zu Phosgen (1a) umgesetzt, wobei in einem anschließenden Schritt in der Isocyanatherstellung (2) das Phosgen (1a) gemeinsam mit einem organischen Amin (23) zum organischen Isocyanat (24) umgesetzt wird. Der in der Isocyanatherstellung (2) freigesetzte Chlorwasserstoff (25) wird der Chlorherstellung (12) zugeführt und der bei der Chlorherstellung anfallende Wasserstoff (12a) wird abgeführt. Der Wasserstoff (12a) kann beispielsweise als Brennstoff (61g) für das Heizelement (61) des Reformerprozesses (6) dienen (nicht in Fig.4 dargestellt).

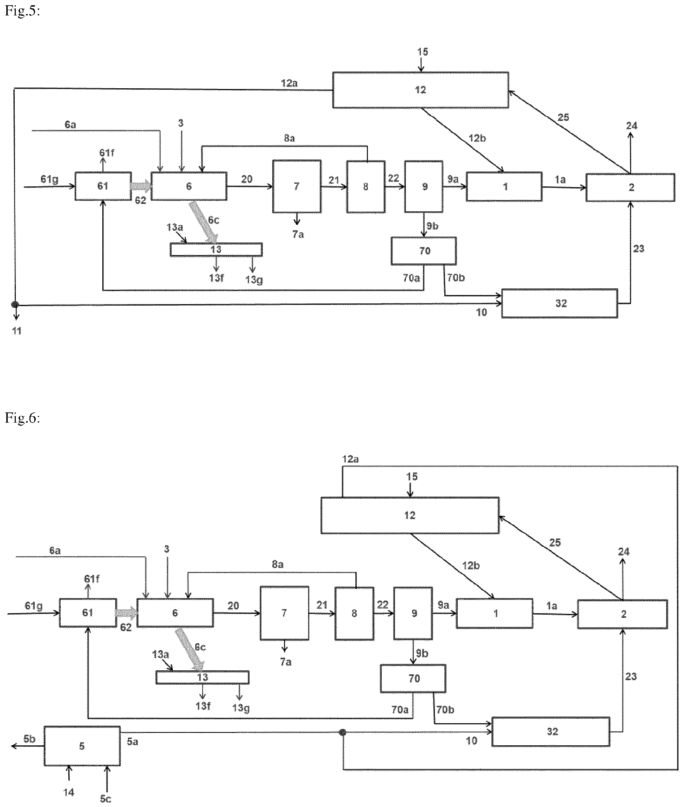

Fig.5 illustriert eine Ausführungsform des in Fig.2 dargestellten Verfahrens, in welchem zusätzlich der bei der Chlorherstellung anfallende Wasserstoff (12a) zumindest teilweise als Wasserstoff (10) als Reaktand für eine Hydrierung organischer Nitroverbindung (32) eingebracht wird, worin dieser mit organischen Nitroverbindungen (nicht dargestellt) zu einem organischen Amin (23) umgesetzt wird. Das resultierende organische Amin (23) wird wiederum der Isocyanatherstellung (2) zugeführt. Teilweise kann der Wasserstoff (12a) als Wasserstoff zur weiteren Verwendung (11) genutzt werden und wird abgeführt. Zusätzlich wird der Hydrierung organischer Nitroverbindung (32) für die besagte Umsetzung zum Isocyanat Wasserstoff aus der Restgasbehandlung (70b) zugeführt.

Fig.6 zeigt eine Ausführungsform des in Fig.2 dargestellten Verfahrens in welchem zusätzlich der bei der Chlorherstellung anfallende Wasserstoff (12a) als Wasserstoff (10) als Reaktand für eine Hydrierung organischer Nitroverbindung (32) eingebracht wird, worin dieser mit organischen Nitroverbindungen (nicht dargestellt) zu einem organischen Amin (23) umgesetzt wird. Das resultierende organische Amin (23) wird wiederum der Isocyanatherstellung (2) zugeführt. Zusätzlich wird der Hydrierung organischer Nitroverbindung (32) für die besagte Umsetzung zum Isocyanat Wasserstoff aus der Restgasbehandlung (70b) und Wasserstoff (5a), der mittels Wasserelektrolyse (5) unter Einsatz elektrischer Energie aus regenerativer Energie (14) und Wasser (5c), zugeführt.

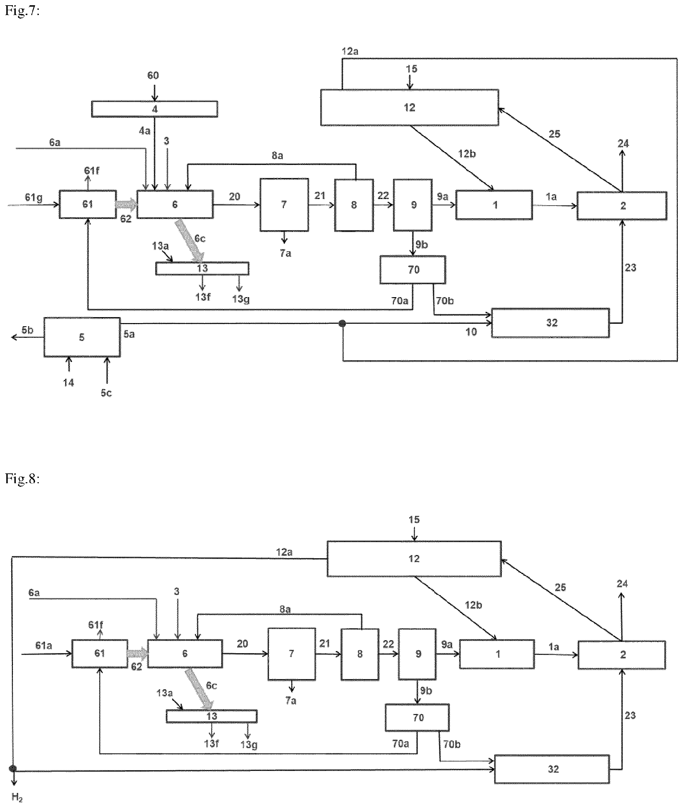

Fig.7 zeigt eine Ausführungsform des in Fig.6 dargestellten Verfahrens, in der zusätzlich zum dem Reformerprozess (6) zugesetzten CO₂ (8a) weiteres CO₂ (4a) aus einer weiteren CO₂-Quelle (60) zugesetzt wird, wobei dieses CO₂ zuvor in einer CO₂ Gasreinigung (4) gereinigt wird. Spendet die weitere CO₂-Quelle (60) bereits gereinigtes CO₂, kann die CO₂ Gasreinigung (4) entfallen.

Die nachfolgenden Aspekte 1 bis 21 stellen eine weitere Ausführungsform der Erfindung dar, wobei die Bezugszeichen in Klammern lediglich klarstellenden Charakter haben und die Ausführungsform nicht auf den Gegenstand der Figuren 1 bis 12 einschränkt:
1. Verfahren für die Herstellung von Kohlenmonoxid aus Methan, Wasserdampf und CO₂ für die Darstellung von Phosgen zur Synthese von organischem Isocyanat, enthaltend zumindest die Schritte
   Synthese von Kohlenmonoxid in einem Reformerprozess (6), worin Methan (6a) und Wasserdampf (3) unter Zusatz von mindestens CO₂ (8a), unter Zuführung von Wärmeenergie bei einer Temperatur von mindestens 500°C zu Kohlenmonoxid-haltigem Produktgas umgesetzt wird,
   Reinigung des aus vorgenannter Synthese erhaltenen Kohlenmonoxid-haltigen Produktgases, mindestens durch Abtrennung von CO₂ (8) und gegebenenfalls zusätzlich durch mindestens eine Abtrennung ausgewählt aus der Abtrennung von Wasser (7), der Abtrennung von Wasserstoff (9) oder einer Kombination davon, unter Erhalt von Kohlenmonoxid;
   Bereitstellung von CO₂ (8a) für den besagten Zusatz zum vorgenannten Reformerprozess (6) zumindest aus besagter Abtrennung von CO₂ (8) des vorgenannten Reinigungsschritts,
   mit der Maßgabe, dass
   i) die zur Synthese von Kohlenmonoxid dem Reformerprozess (6) zugeführte Wärmeenergie durch mindestens eine Methode bereitgestellt wird, ausgewählt aus (i) der Verbrennung von Brennstoff, der mittels regenerativer Energie erzeugten Wasserstoff enthält, (ii) der Verbrennung von Brennstoff, der Methan aus biologischer Quelle enthält, (iii) Umwandlung von aus regenerativer Energie erzeugter, elektrischer Energie (6c) in Wärme; oder
   ii) für die Bereitgestellung des der Synthese des vorgenannten Reformerprozesses (6) zugesetzten CO₂ zusätzlich CO₂ (4a) aus einer weiteren CO₂-Quelle (60) herangezogen wird; oder
   iii) eine Kombination der vorgenannten Möglichkeiten i) und ii) gewählt wird.
2. Verfahren gemäß Aspekt 1, dadurch gekennzeichnet, dass als Methan-Quelle zur Bereitstellung des Methans (6a) für den Reformerprozess (6) Methan aus fossiler Quelle, Methan aus biologischer Quelle oder Gemische daraus, insbesondere Methan aus biologischer Quelle, dient.
3. Verfahren nach Aspekt 1 oder 2, dadurch gekennzeichnet, dass die als regenerative Energie wahlweise Windkraft, Sonnenenergie, Wasserkraft oder Mischungen daraus, eingesetzt wurde.
4. Verfahren gemäß einem der Aspekte 1 bis 3, dadurch gekennzeichnet, dass der für die Verbrennung von Wasserstoff-haltigem Brennstoff eingesetzte Wasserstoff durch mindestens ein Elektrolyseverfahren bereitgestellt wird, ausgewählt aus der Wasserelektrolyse (5) von Wasser (5c) zu Wasserstoff (5a) und Sauerstoff (5b), der Chlor-Alkali-Elektrolyse, der Salzsäure-Elektrolyse, wobei die für das Elektrolyseverfahren genutzte elektrische Energie mittels regenerativer Energie (15) erzeugt wird.
5. Verfahren gemäß einem der vorangehenden Aspekte, dadurch gekennzeichnet, dass die Synthese im Reformerprozess (6) bei einer Temperatur von mindestens 700°C, bevorzugt in einem Bereich von 800 bis 1100 °C, besonders bevorzugt in einem Bereich von 850 bis 1000°C, abläuft.
6. Verfahren gemäß einem der vorangehenden Aspekte, dadurch gekennzeichnet, dass die Synthese im Reformerprozess (6) bei einem absoluten Druck in einem Bereich von 10 bis 40 bar, bevorzugt von 15 bis 35 bar, besonders bevorzugt 18 bis 25 bar, abläuft.
7. Verfahren gemäß einem der vorangehenden Aspekte, dadurch gekennzeichnet, dass die Synthese im Reformerprozess (6) unter Einsatz eines an einer festen Phase geträgerten Katalysators stattfindet.
8. Verfahren gemäß einem der vorangehenden Aspekte, dadurch gekennzeichnet, dass der Reformerprozess (6) mindestens zwei Schritte umfasst
   a) Umsetzung von zumindest Methan (6a) und Wasserdampf zu einem Gemisch, mindestens enthaltend Kohlenmonoxid und Wasserstoff
   b) Umsetzung zumindest des besagten Gemisches aus (a) unter Zusatz von CO₂ zu Kohlenmonoxid-haltigem Produktgas.
9. Verfahren gemäß einem der vorangehenden Aspekte, dadurch gekennzeichnet, dass die besagte Abtrennung von Wasser (7) erfolgt und bevorzugt das dabei abgetrennte Wasser (7a) in eine Wasserelektrolyse (5) rezykliert wird.
11. Verfahren gemäß einem der vorangehenden Aspekte, dadurch gekennzeichnet, dass nach der durchgeführten Abtrennung von Wasserstoff (9),insbesondere unter Verwendung einer Coldbox, der aus dem Restgas (9b) nach weiterer Restgasbehandlung (70) hervorgehende Wasserstoff (70b) in eine Hydrierung von organischer Nitroverbindung (30) zur Herstellung von organischem Amin als Rohstoff für organisches Isocyanat eingespeist oder das Restgas der H₂-CO Trennung (9b) als Brennstoff zur Bereitstellung von Wärmeenergie des Reformerprozesses (6) in der Brennkammer des Heizelementes oder zur Wasserdampf-Erzeugung genutzt und/oder das nach weiterer Restgasbehandlung (70) erhaltene Restgas der Restgasbehandlung (70a) als Brennstoff zur Bereitstellung von Wärmeenergie des Reformerprozesses in der Brennkammer des Heizelementes (61) oder als erhaltenes Restgas der Restgasbehandlung (70c) zur Wasserdampf-Erzeugung genutzt.
12. Verfahren gemäß einem der vorangehenden Aspekte, dadurch gekennzeichnet, dass eine Synthese (1) von Phosgen (1a) aus zumindest Kohlenmonoxid (9a) und Chlor (12b) erfolgt.
13. Verfahren gemäß Aspekt 12, dadurch gekennzeichnet, dass eine Umsetzung (2) von Phosgen (1a) mit mindestens einem organischen Amin (23) zu mindestens einer organischen Isocyanat-Verbindung (24) und Chlorwasserstoff (25) erfolgt.
14. Verfahren nach einem der Aspekte 12 oder 13, dadurch gekennzeichnet, dass das verbliebene Kohlenmonoxid (9a) aus der Abtrenneinheit (9) in die Phosgensynthese (1) eingespeist wird.
15. Verfahren nach einem der vorangehenden Aspekte, dadurch gekennzeichnet, dass Wasserstoff (5a) aus der Wasserelektrolyse (5) in die Hydrierung von organischer Nitroverbindung (32) für die Herstellung von organischem Amin eingespeist wird.
16. Verfahren nach einem der Aspekte 13 oder 14, dadurch gekennzeichnet, dass die Abtrennung und Reinigung des in der Isocyanat-Herstellung (2) gebildeten Chlorwasserstoffs (25) und eine anschließende oxidative Umsetzung (12) des Chlorwasserstoffs, ausgewählt aus
   - einer Umsetzung von Chlorwasserstoff in einer thermokatalytischen Gasphasenoxidation mit Sauerstoff zu Chlor und Wasser unter ggf. Nutzung von Sauerstoff aus der Wasserelektrolyse (5),
   - einer Umsetzung von Chlorwasserstoff mit Wasser zu Salzsäure und anschließender elektrochemischer Oxidation der Salzsäure zu Chlor und ggf. Wasserstoff,
   erfolgt und an die Umsetzung (12) eine Zuführung des gebildeten Chlors (12b) in die Phosgensynthese (1) anschließt, gegebenenfalls unter Zuführung einer zusätzlichen Menge Chlor in die Phosgensynthese (1), die aus einer Chloralkali-Elektrolyse stammt.
17. Verfahren gemäß einem der Aspekte 5, 10, 15 oder 16, dadurch gekennzeichnet, dass die Wasserelektrolyse (5) und/oder die elektrochemische Oxidation (12) unter Verwendung von aus regenerativer Energie erzeugtem elektrischem Strom durchgeführt wird, insbesondere aus regenerativer Energie in Form von Windkraft, Sonnenenergie oder Wasserkraft.
18. Verfahren gemäß einem der vorangehenden Aspekte, dadurch gekennzeichnet, dass mindestens 80 % der für das gesamte Verfahren eingesetzten Energie mittels aus regenerativer Energie erzeugten elektrischen Stroms (6c) durchgeführt wird, insbesondere elektrischen Stroms (6c) wahlweise erhalten durch Nutzung von Windkraft, Sonnenenergie oder Wasserkraft.
19. Verfahren gemäß einem der vorangehenden Aspekte, dadurch gekennzeichnet, dass die in Reformerprozess (6) zugeführte Wärmeenergie zumindest aus dem mittels Wasserelektrolyse (5) erzeugten Wasserstoff (5a) oder Sauerstoff (5b) erzeugt wird.
20. Vorrichtung für die Synthese von Kohlenmonoxid unter Bereitstellung von Kohlenmonoxid für die Herstellung von Phosgen für die Synthese von organischem Isocyanat umfasst mindestens folgende Vorrichtungsteile
   - mindestens einen Reformer, enthaltend mindestens einen Reaktionsraum, mindestens ein Heizelement als eine Vorrichtung zur Zuführung von Wärmeenergie zum Reaktionsraum, sowie mindestens einen Einlass für die Einbringung von Methan, Wasserdampf und CO₂ in den Reaktionsraum und mindestens einen Auslass für das Kohlenmonoxid-haltige Produktgas aus dem Reformer;
   - mindestens eine Vorrichtung zur Abtrennung von Wasser, die zumindest einen Einlass aufweist, der in Fluidverbindung mit dem Auslass für das Kohlenmonoxid-haltige Produktgas aus dem Reformer steht und mindestens einen Auslass für das abgetrennte Wasser und mindestens einen Auslass für das getrocknete, Kohlenmonoxid-haltigen Produktgas;
   - mindestens eine Vorrichtung zur Abtrennung von CO₂, die zumindest einen Einlass aufweist, der in Fluidverbindung mit dem Auslass für das getrocknete, Kohlenmonoxid-haltige Produktgas aus der Vorrichtung zur Abtrennung von Wasser steht und mindestens einen Auslass für das abgetrennte CO₂, der in Fluidverbindung mit einem Einlass für CO₂ des Reformers steht und mindestens einen Auslass für das vorgereinigte, Kohlenmonoxid-haltige Produktgas,;
   - mindestens eine Vorrichtung zur Trennung von H₂-CO, die zumindest einen Einlass aufweist, der in Fluidverbindung mit dem Auslass für das vorgereinigte, Kohlenmonoxid-haltige Produktgas aus der Vorrichtung zur Abtrennung von CO₂ steht und mindestens einen Auslass für das Kohlenmonoxid und mindestens einen Auslass für das Restgas der H₂-CO Trennung;
   - mindestens eine Vorrichtung zur Herstellung von Phosgen, die mindestens einen Einlass für Kohlenmonoxid aufweist, der in Fluidverbindung mit dem für das Kohlenmonoxid vorgesehenen Auslass der Vorrichtung zur Trennung von H₂-CO in Fluidverbindung steht, sowie mindestens einen Einlass für Chlor und mindestens einen Auslass für Phosgen enthält;
   - mindestens eine Vorrichtung zur Herstellung von Chlor, die mindestens einen Einlass für Chlorwasserstoff aufweist, sowie mindestens einen Auslass für Wasserstoff und mindestens einen Auslass für Chlor enthält, der mit einem Einlass für Chlor von der Vorrichtung zur Herstellung von Phosgen in Fluidverbindung steht;
      mit der Maßgabe, dass
      i) das Heizelement des Reformers in Fluidverbindung mit mindestens einer Quelle, ausgewählt aus einer Quelle für aus regenerativer Energie bereitgestelltem Wasserstoff als Brennstoff, einer Quelle für aus biologischer Quelle bereitgestelltem Methan als Brennstoff, steht oder das Heizelement an eine Quelle zur Bereitstellung von elektrischer Energie aus regenerativer Energie für die Umwandlung in Wärmeenergie angeschlossen ist; und/oder
      ii) ein Einlass des Reaktors in Fluidverbindung mit einer weiteren CO₂-Quelle zur Einbringung von zusätzlichem CO₂ in den Reaktionsraum steht, herangezogen wird.
21. Verwendung einer Vorrichtung, die für die Synthese von Kohlenmonoxid (9a) in einem Reformerprozess (6) ausgestaltet ist, worin Methan mit Wasserdampf unter Zusatz von mindestens CO₂ (4a), unter Zuführung von Wärmeenergie bei einer Temperatur von mindestens 500°C zu Kohlenmonoxid-haltigem Produktgas umgesetzt wird, und
   die für die Reinigung des aus vorgenannter Synthese erhaltenen Kohlenmonoxid-haltigen Produktgases ausgestaltet ist, mindestens durch Abtrennung von Wasser, durch Abtrennung von CO₂, durch Abtrennung von Wasserstoff oder durch eine Kombination von mindestens zwei der vorgenannten Abtrennungen in jeweils eine entsprechenden Abtrenneinheit, unter Erhalt von Kohlenmonoxid (9a),
   zur Bereitstellung von Kohlenmonoxid (9a) für die Herstellung von organischem Isocyanat, nach einem Verfahren, umfassend zumindest die Schritte
   Synthese (1) von Phosgen (1a) aus zumindest Kohlenmonoxid (9a) und Chlor (12b),
   Umsetzung (2) von Phosgen (1a) mit mindestens einem organischen Amin (23) zu mindestens einer organischen Isocyanat-Verbindung (24) und Chlorwasserstoff (25).
22. Verwendung nach Aspekt 21, wobei die Vorrichtung eine Vorrichtung gemäß Aspekt 20 ist.

### Beispiele

Die nachfolgenden Beispiele beziehen sich auf die Herstellung von Kohlenmonoxid eingebunden in die Synthese von TDI, das erfindungsgemäße Verfahren kann ebenso auf die Herstellung anderer Isocyanate angewandt werden.

Tabellarische Übersicht der Beispiele, für jeweils immer eine Produktion von 11,4 t/h CO sowie nötigenfalls eine Normierung der CO₂-Beiträge auf 6,1 MW Wasserdampf:

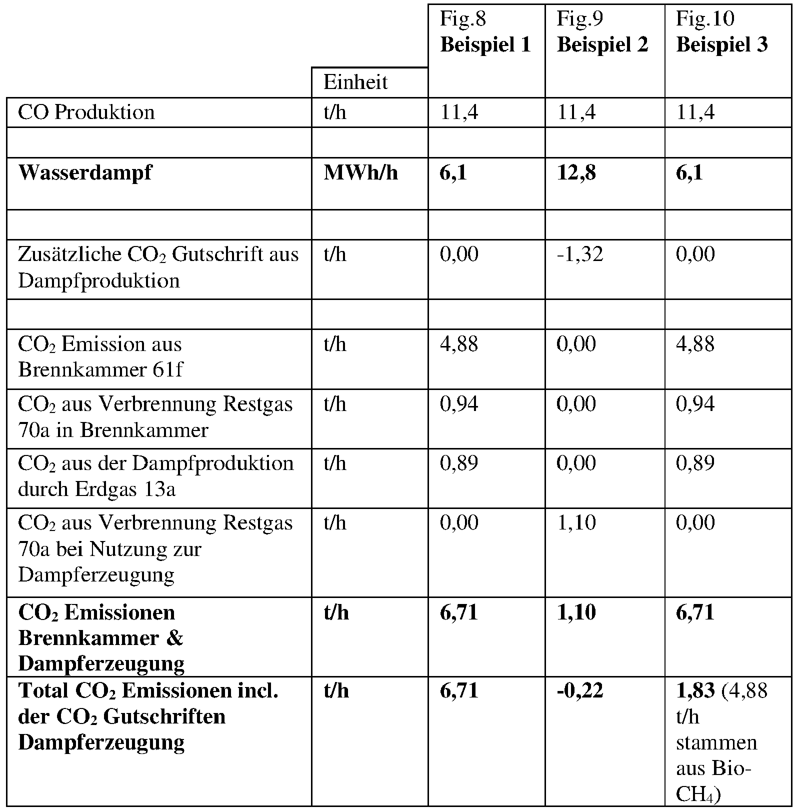

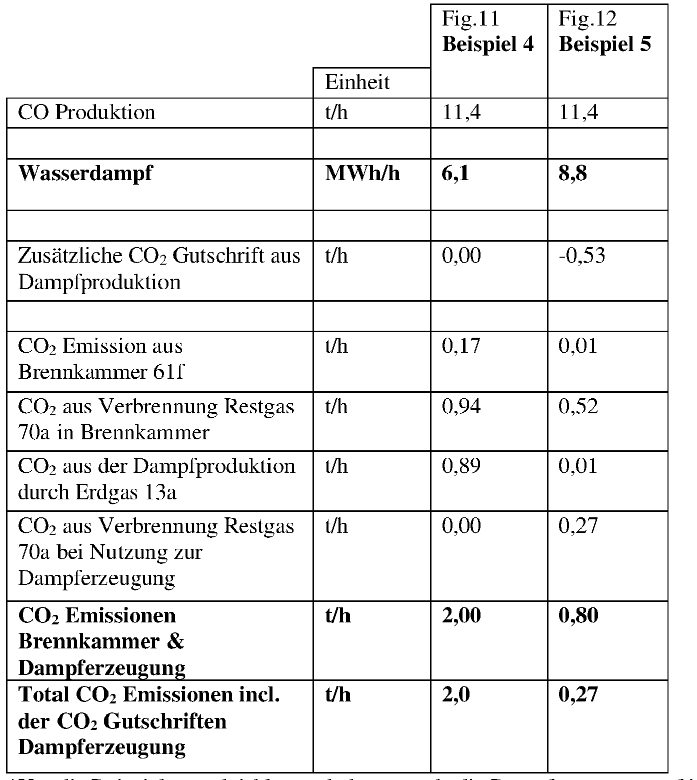

*Um die Beispiele vergleichbar zu halten, wurde die Dampferzeugung auf jeweils 6,1 MWh berechnet. 6,1MW entsprechen dem Beispiel 1, dem Stand der Technik. Wird mehr Dampf erzeugt, so wird die CO₂ Emissionen aus der Dampferzeugung negativ gerechnet. Hierbei wird bei mehr erzeugtem Dampf eine entsprechende CO₂-Gutschrift eingerechnet auf Basis von 0,0099 MWh/m³ Erdgas entsprechend 2,9 MWh Dampf und 0,57 t CO₂ Emission.

Zusammenfassung: Gegenüber dem Stand der Technik führt der Einsatz von elektrischer Energie zum Heizen des Heizelementes (61) oder der Einsatz von Wasserstoff zum Heizen der Brennkammer zu niedrigeren CO₂ Emissionen. Wird Bio-Methan eingesetzt, so entstehen zwar gleiche Mengen CO₂ Emissionen, jedoch können die Anteile von Bio-Methan als nachhaltig betrachtet werden, da bei der Herstellung von Bio-Methan CO₂ bei der Erzeugung der Bio-Masse verbraucht wird, hierdurch ergeben sich insgesamt betrachtet geringere CO₂-Emissionen. Die geringsten CO₂ Emissionen können dadurch erhalten werden, dass Bio-Methan sowohl für die Brennkammer des Heizelementes (61), zur ggf. Dampferzeugung als auch als Edukt für den Reformerprozess eingesetzt werden. Weiterhin können durch die elektrische Beheizung großen Mengen CO₂-Emissionen vermieden werden als auch durch den zusätzlichen Einsatz von CO₂ aus einer externen Quelle, wobei die intern im Gesamtverfahren zu recyclierenden CO₂-Mengen (8a) größer werden.

### Tabellarische Übersicht der Beispiele, in denen jeweils 11,4 t/h CO hergestellt werden

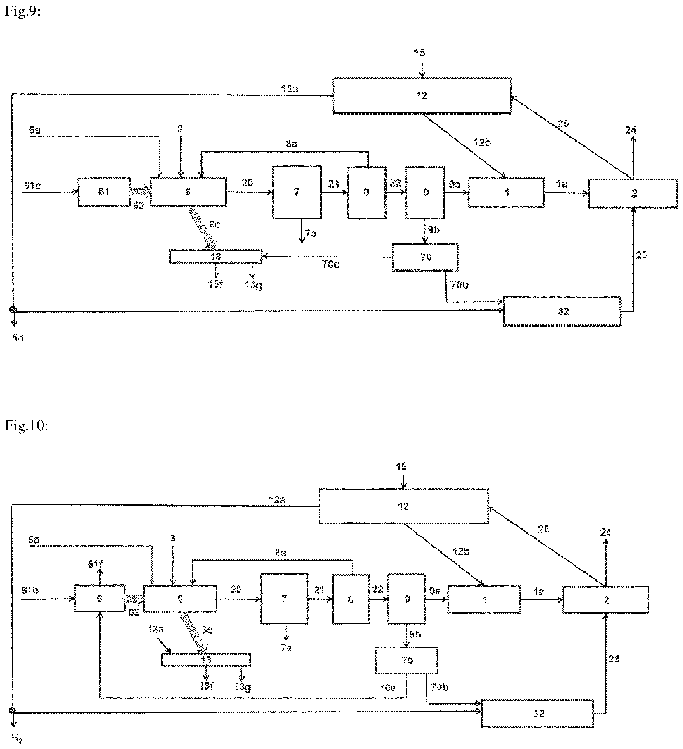

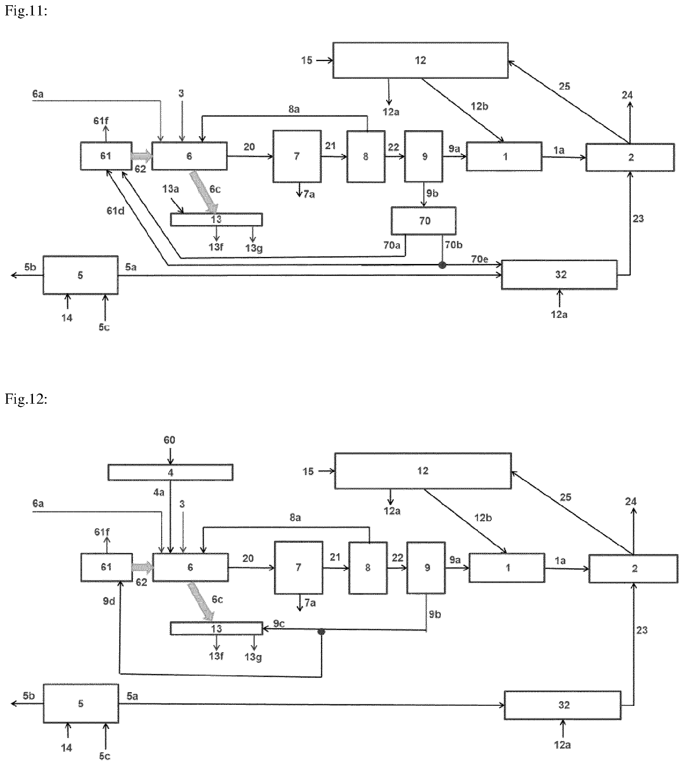

- Beispiel 1 - Fig. 8: Stand der Technik
- Beispiel 2 - Fig. 9: erfindungsgemäß.: Heizung mittels regenerativer elektrischer Energie
- Beispiel 3 - Fig. 10: erfindungsgemäß: Heizung mittels Bio-Erdgas
- Beispiel 4 - Fig. 11: erfindungsgemäß: Heizung mittels grünem Wasserstoff
- Beispiel 5 - Fig. 12: erfindungsgemäß: Zuführung von CO₂ aus externer Quelle

Das erfindungsgemäße Verfahren soll am Beispiel der Herstellung von TDI dargestellt werden. Das hergestellte TDI wird ausschließlich aus CO aus fossiler Quelle hergestellt. Das TDI besitzt 9 Kohlenstoffatome, wobei 2 Kohlenstoffatome die Isocyanatgruppe bilden. Diese Kohlenstoffatome stammen aus dem Kohlenstoff des Kohlenmonoxid. Somit beträgt der Anteil an fossilem Kohlenstoff im TDI 22,2 Gew.%. Durch Verringerung der CO₂-Emissionen kann ein nachhaltigeres TDI hergestellt werden. Die Beispiele stellen eine Möglichkeit bzgl. der Verwendung des Restgases aus der H₂-CO-Trenneinheit abgetrennten Gases dar. Das Restgas kann weiter aufgetrennt werden, so dass reiner Wasserstoff erhalten werden kann oder es kann thermisch zur Dampferzeugung (13) oder in der Brennkammer des Heizelementes 61 des Reformers genutzt werden. Wird Wasserstoff abgetrennt, kann dieser sowohl zum Heizen der Brennkammer oder zur Dampferzeugung als auch zur Hydrierung von Nitroaromaten eingesetzt werden. Ebenfalls kann Wasserstoff aus dem HCl-Recycling der Isocyanat-Herstellung entsprechend eingesetzt werden. Fehlende Wasserstoffmengen können über eine Wasserelektrolyse bereitgestellt werden. Der bei der Wasserelektrolyse entstehende Sauerstoff kann bei der Chlor-Herstellung mittels HCl-ODC oder NaCl-ODC Technologie oder bei der HCl-Gasphasenoxidation eingesetzt werden. Grundsätzlich kann durch Nutzung von Bio-Erdgas (Methan aus biologischer Quelle) im Reformerprozess die CO₂-Emission noch weiter erniedrigt werden.

### Beispiel 1 - Stand der Technik (Fig.8) zur Herstellung von Toluol-diisocyanat (TDI)

Einem Reformerprozess (6) wurden 6,92 t/h Erdgas, mit einer Zusammensetzung von mind. 98% Methan und mind. 1% weiteren Nebenbestandteilen wie Ethan und höheren Kohlenwasserstoffen, 19,0 t/h Wasserdampf, sowie 3,76 t/h CO₂ aus der CO₂ Abtrenneinheit (8) eingebracht. Der Brennkammer des Heizelementes (61) des Reformerprozesses (6) wurden 1,72 t/h Erdgas sowie das 0,92 t/h Restgas (70a) aus der Restgasbehandlung (70) bestehend aus 0,60 t/h CO, 0,26 t/h H₂ und 0,06 t/h Methan zugeführt. Der Reformerprozess wurde bei einer Temperatur von 950°C und bei einem Druck von 20 bar durchgeführt.

Aus dem Reformerprozess erhaltene Produktgasgemisch (20) überwiegend bestehend aus 12,0 t/h CO, 11,32 t/h H₂O, 3,76 t/h CO₂, 2,58 t/h H₂ und 0,06 t/h Methan wurde einer H₂O Abtrenneinheit (7) zugeführt, in der 11,32 t/h Wasser aus dem Gasgemisch (20) abgetrennt wurden. Das verbleibende Gasgemisch (21) aus der H₂O-Abtrennung (7) wurde einer CO₂ Abtrenneinheit (8) zugeführt, in der 3,76 t/h CO₂ abgetrennt und dem Reformerprozess (6) wieder zugeführt wurden. Die CO₂ Abtrennung erfolgt mittels Aminwäsche. Das von CO₂ befreite Gas (22) bestehend aus 12,0 t/h CO und 2,58 t/h H₂ sowie 0,06 t/h Methan wurde der H₂-CO Trenneinheit (9) zugeführt. Für die H₂-CO Trennung wurde eine sogenannte Cold-box eingesetzt, bei der das H₂-CO Gasgemisch abgekühlt und Wasserstoff und CO getrennt wurden. Dabei wurde eine Restgas mit (9a) 3,24 t/h bestehend aus 0,60 t/h CO, 2,58 t/h H₂ und 0,06 t/h Methan abgetrennt und einer Restgasbehandlung (70) zugeführt. Die Restgasbehandlung (70) ist eine nach dem Druckwechseladsorptions-Prinzip (PSA) arbeitende Trenneinheit. Aus der Restgasbehandlung 70 wurde ein Restgas 70a mit 0,92 t/h bestehend aus 0,6 t/h CO und 0,26 t/h H₂ sowie 0,06 t/h Methan der Brennkammer des Heizelementes (61) zugeführt. Die aus der Restgaseinheit abgetrennter H₂ mit 2,32 t/h wurde der Hydrierung der Nitroaromaten (32) zugeführt. Aus der H₂-CO Trenneinheit (9) wurden 11,4 t/h CO der Phosgensynthese zugeführt.

18,72 t/h Toluol wurden in einer Dinitrotoluol-Herstellung (30) mit 25,65 t/h Salpetersäure, gerechnet als HNO₃, zu 37,04 t/h Dinitrotoluol umgesetzt. Das Dinitrotoluol wurde anschließend in einer Hydrierungsstufe (32) mit 2,44 t/h Wasserstoff umgesetzt. Dabei wurden 2,32 t/h H₂ aus der CO-H₂-Trenneinheit eingesetzt und 0,14 t/h aus einer mit regenerativ betriebener Energie betriebenen HCl-Diaphragmaelektrolyse (Salzsäureelektrolyse) der Chlor-Herstellung (12). Dabei entstehen 24,63 t/h Toluol-Diamin welches anschließend in einer Isocyanat-Herstellung (2) mit Phosgen zu Isocyanat umgesetzt wurde.

Das Phosgen wurde in der Phosgen-Synthese (1) hergestellt aus 11,4 t/h CO und 28,90 t/h Chlor, welches aus einer Chlor-Herstellung (12) durch eine Salzsäureelektrolyse erzeugt wurde.

Aus der Phosgensysnthese (1) wurden 40,3 t/h Phosgen entnommen und in einer Isocyanat-Herstellung (2) mit 24,63 t/h Toluol-Diamin zu 35,41 t/h Toluol-diisocyanat umgesetzt. Das erhaltene Toluol-diisocyanat kann in üblicher Weise mit Polyetherpolyolen oder Polyesterpolyolen in einer Polyurethan-Synthese zu Polyurethan-Material umgesetzt werden.

Das bei der Isocyanat-Herstellung anfallende HCl-Gas (25) mit einer Menge von 29,71 t/h wurde nach Reinigung über eine Tieftemperaturdestillation in Wasser absorbiert und die entstehende Salzsäure nochmals über Aktivkohle gereinigt und in einer Salzsäureelektrolyse zu 28,9 t/h Chlor und 0,81 t/h Wasserstoff umgesetzt. Der 0,14 t/h des Wasserstoffs wurden zusammen mit dem H₂ aus der Restgasbehandlung (70) der Hydrierung der Nitroaromaten (32) zugeführt. Das erhaltene Chlor (12b) wurde der Phosgen-Synthese (1) zugeführt.

Aus dem Reformerprozess (6) konnte Dampf (13g) mit einem Energieinhalt von 6,1 MWh/h erhalten werden. Hierbei wurde der Dampferzeugung (13) zusätzlich Erdgas (13a) mit 0,32 t/h zugeführt.

Aus dem Prozess wurden folgende CO₂-Emissionen erzeugt:

| CO₂-Quelle | t/h CO₂ |
|---|---|
| Brennkammer - Verbrennung Erdgas 61a | 4,88 |
| Brennkammer - Verbrennung Restgas 70a | 0,94 |
| Dampferzeugung Verbrennung Erdgas 13a | 0,89 |
| **Gesamte CO₂ Emissionen** | **6,71** |

### Beispiel 2: Erfindungsgemäßes Verfahren (Fig. 9) - Verringerung des Einsatzes von fossilem Erdgas durch Einbringung von elektrischer Energie zum Heizen des Heizelementes bei der Herstellung von Toluol-diisocyanat (TDI)

Einem Reformerprozess (6) wurden 6,92 t/h Erdgas (6a), mit einer Zusammensetzung von mind. 98% Methan und mind. 1% weiteren Nebenbestandteilen wie Ethan und höheren Kohlenwasserstoffen, 19,0 t/h Wasserdampf, sowie 3,76 t/h CO₂ (8a) aus der CO₂ Abtrenneinheit (8) eingebracht. Weiterhin wurde durch elektrische Beheizung die notwendige Energie für den Reformerprozess (6) eingebracht. Hierbei wurde das Heizelement (61) mittels elektrischer betriebener Heizwendel beheizt. Eine elektrische Heizleistung (61c) von 34,9 MWh wurde je Stunde zugeführt. Der Reformerprozess wurde bei einer Temperatur von 950°C und bei einem Druck von 20 bar durchgeführt.

Aus dem Reformerprozess erhaltene Produktgasgemisch (20) überwiegend bestehend aus 12,0 t/h CO, 11,32 t/h H₂O, 3,76 t/h CO₂, 2,58 t/h H₂ und 0,06 t/h Methan wurde einer H₂O Abtrenneinheit (7) zugeführt, in der 11,32 t/h Wasser (7a) aus dem Gasgemisch (20) abgetrennt wurden. Das verbleibende Gasgemisch (21) aus der H₂O-Abtrennung (7) wurde einer CO₂ Abtrenneinheit (8) zugeführt, in der 3,76 t/h CO₂ (8a) abgetrennt und dem Reformerprozess (6) wieder zugeführt wurden. Die CO₂ Abtrennung erfolgt mittels Aminwäsche. Das von CO₂ befreite Gas (22) bestehend aus 12,0 t/h CO und 2,58 t/h H₂ sowie 0,06 t/h Methan wurde der H₂-CO Trenneinheit (9) zugeführt. Für die H₂-CO Trennung wurde eine sogenannte Cold-box eingesetzt, bei der das H₂-CO Gasgemisch abgekühlt und Wasserstoff und CO getrennt wurden. Dabei wurde ein Restgas (9b) einer Restgasbehandlung (70) zugeführt. Aus der Restgasbehandlung wurde 2,32 t/h Wasserstoff (70b) abgetrennt und der Hydrierung der Nitroaromaten (32) zugeführt. Das verbleibende Restgas (70c) mit 0,92 t/h bestehend aus 0,26 t/h H₂, 0,60 t/h CO und 0,06 t/h Methan wurde der Dampferzeugung (13) zugeführt.

Der aus der Restgasabtrennung (70) abgetrennte Wasserstoff (70b) von 2,32 t/h H₂ wurde zusammen mit 0,14 t/h H₂ (12a)aus der Salzsäureelektrolyse der Chlor-Herstellung (12) der Hydrierung der Nitroaromaten (32) zugeführt wurde. Der restliche H₂ aus der Salzsäureelektrolyse (12a) kann einer weiteren Verwendung zugeführt werden.

Aus der H₂-CO Trenneinheit (9) wurden 11,4 t/h CO der Phosgensynthese zugeführt.

18,72 t/h Toluol wurden in einer Dinitrotoluol-Herstellung (30) mit 25,65 t/h Salpetersäure, gerechnet als HNO₃, zu 37,04 t/h Dinitrotoluol umgesetzt. Das Dinitrotoluol wurde anschließend in einer Hydrierungsstufe (32) mit 2,44 t/h Wasserstoff aus der Restgasabtrennung (70) und der Salzsäureelektrolyse (12) umgesetzt. Dabei entstehen 24,63 t/h Toluol-Diamin welches anschließend in einer Isocyanat-Herstellung (2) mit Phosgen zu Isocyanat umgesetzt wurde.

Das Phosgen wurde in der Phosgen-Synthese (1) hergestellt aus 11,4 t/h CO und 28,90 t/h Chlor, welches aus einer Chlor-Herstellung (12) durch eine Salzsäureelektrolyse erzeugt wurde.

Aus der Phosgensynthese (1) wurden 40,3 t/h Phosgen entnommen und in einer Isocyanat-Herstellung (2) mit 24,63 t/h Toluol-Diamin zu 35,41 t/h Toluol-diisocyanat umgesetzt. Das erhaltene Toluol-diisocyanat kann in üblicher Weise mit Polyetherpolyolen oder Polyesterpolyolen in einer Polyurethan-Synthese zu Polyurethan-Material umgesetzt werden.

Das bei der Isocyanat-Herstellung anfallende HCl-Gas (25) mit einer Menge von 29,71 t/h wurde nach Reinigung über eine Tieftemperaturdestillation in Wasser absorbiert und die entstehende Salzsäure nochmals über Aktivkohle gereinigt und in einer Salzsäureelektrolyse zu 28,9 t/h Chlor und 0,81 t/h Wasserstoff umgesetzt. Eine Verfahrensvariation ist, dass der Wasserstoff aus der Salzsäureelektrolyse (12) zusammen mit dem H₂ aus der Restgasbehandlung (70) der Hydrierung der Nitroaromaten zugeführt wurde, wobei der H₂-Bedarf bei 2,44 t/h beträgt.

Aus dem Reformerprozess (6) kann Dampf (13g) mit einem Energieinhalt von 12,8 MWh/h erhalten werden.

Aus dem Prozess wurden folgende CO₂-Emissionen erzeugt:

| CO₂-Quelle | t/h CO₂ |
|---|---|
| Dampferzeugung - Verbrennung Restgas 70a | 1,10 |
| CO₂ Gutschrift aus der Dampferzeugung | -1,32 |
| **Gesamte CO₂ Emissionen** | **-0,22** |

### Beispiel 3: Erfindungsgemäßes Verfahren Fig. 10 - Herstellung von emissionsarmen Toluol-diisocyanat (TDI), Verringerung des Einsatzes von fossilem Erdgas durch Nutzung von Bio-Methan zur Beheizung der Brennkammer

Einem Reformerprozess (6) wurden 6,92 t/h Erdgas, mit einer Zusammensetzung von mind. 98% Methan und mind. 1% weiteren Nebenbestandteilen wie Ethan und höheren Kohlenwasserstoffen, 19,0 t/h Wasserdampf, sowie 3,76 t/h CO₂ aus der CO₂ Abtrenneinheit (8) eingebracht. Als Brenngas die Brennkammer des Heizelementes (61) wurde Bio-Methan (61b) eingesetzt. Der Brennkammer des Heizelementes (61) wurden 1,72 t/h Bio-Methan (61b) sowie das 0,92 t/h Restgas (70a) aus der Restgasbehandlung (70) bestehend aus 0,60 t/h CO, 0,26 t/h H₂ und 0,06 t/h Methan zugeführt. Der Reformerprozess wurde bei einer Temperatur von 950°C und bei einem Druck von 20 bar durchgeführt.

Aus dem Reformerprozess erhaltene Produktgasgemisch (20) überwiegend bestehend aus 12,0 t/h CO, 11,32 t/h H₂O, 3,76 t/h CO₂, 2,58 t/h H₂ und 0,06 t/h Methan wurde einer H₂O Abtrenneinheit (7) zugeführt, in der 11,32 t/h Wasser aus dem Gasgemisch (20) abgetrennt wurden. Das verbleibende Gasgemisch (21) aus der H₂O-Abtrennung (7) wurde einer CO₂ Abtrenneinheit (8) zugeführt, in der 3,76 t/h CO₂ abgetrennt und dem Reformerprozess (6) wieder zugeführt wurden. Die CO₂ Abtrennung erfolgt mittels Aminwäsche. Das von CO₂ befreite Gas (22) bestehend aus 12,0 t/h CO und 2,58 t/h H₂ sowie 0,06 t/h Methan wurde der H₂-CO Trenneinheit (9) zugeführt. Für die H₂-CO Trennung wurde eine sogenannte Cold-box eingesetzt, bei der das H₂-CO Gasgemisch abgekühlt und Wasserstoff und CO getrennt wurden. Dabei wurde ein Restgas (9b) erhalten, welches der Restgasbehandlung (70) zugeführt wurde. Aus der Restgasbehandlung wurden 2,32 t/h H2 (70b) der Hydrierung der Nitroaromaten (32) zugeführt. Das verbleibende Restgas (70a) der Restgasbehandlung (70) bestehend aus 0,26 t/h H₂, 0,60 t/h CO und 0,06 t/h Methan wurde der Brennkammer des Heizelementes (61) zugeführt. Der aus der Restgasbehandlung (70) abgetrennte Wasserstoff von 2,32 t/h H₂ wurde zusammen mit 0,14 t/h H₂ aus der Salzsäurelektrolyse der Chlor-Herstellung (12) der Hydrierung der Nitroaromaten (32) zugeführt.

Aus der H₂-CO Trenneinheit (9) wurden 11,4 t/h CO der Phosgensynthese zugeführt.

18,72 t/h Toluol wurden in einer Dinitrotoluol-Herstellung (30) mit 25,65 t/h Salpetersäure, gerechnet als HNO₃, zu 37,04 t/h Dinitrotoluol umgesetzt. Das Dinitrotoluol wurde anschließend in einer Hydrierungsstufe (32) mit 2,44 t/h Wasserstoff aus einer mit regenerativ betriebener Enerie betriebenen Wasserelektrolyse (5) umgesetzt. Dabei entstehen 24,63 t/h Toluol-Diamin welches anschließend in einer Isocyanat-Herstellung (2) mit Phosgen zu Isocyanat umgesetzt wurde.

Das Phosgen wurde in der Phosgen-Synthese (1) hergestellt aus 11,4 t/h CO und 28,90 t/h Chlor, welches aus einer Chlor-Herstellung (12) durch eine Salzsäureelektrolyse erzeugt wurde.

Aus der Phosgensynthese (1) wurden 40,3 t/h Phosgen entnommen und in einer Isocyanat-Herstellung (2) mit 24,63 t/h Toluol-Diamin zu 35,41 t/h Toluol-diisocyanat umgesetzt. Das erhaltene Toluol-diisocyanat kann in üblicher Weise mit Polyetherpolyolen oder Polyesterpolyolen in einer Polyurethan-Synthese zu Polyurethan-Material umgesetzt werden.

Das bei der Isocyanat-Herstellung anfallende HCl-Gas (25) mit einer Menge von 29,71 t/h wurde nach Reinigung über eine Tieftemperaturdestillation in Wasser absorbiert und die entstehende Salzsäure nochmals über Aktivkohle gereinigt und in einer Salzsäureelektrolyse zu 28,9 t/h Chlor und 0,81 t/h Wasserstoff umgesetzt.

Aus dem Reformer (6) kann Dampf mit einem Energieinhalt von 6,1 MWh/h erhalten wurden. Hierzu wurde bei der Dampferzeugung zusätzlich Erdgas (13a) mit 0,32 t/h zugeführt.

| CO₂-Quelle | t/h CO₂ |
|---|---|
| Brennkammer - Verbrennung Bio-Methan 61b - CO₂ Emissionen von 4,9 t/h CO₂ sind klimaneutral | 0 (klimaneutral) |
| Brennkammer - Verbrennung Restgas 70a | 0,94 |
| Dampferzeugung - Verbrennung Erdgas 13a | 0,89 |
| **Gesamte CO₂ Emissionen** | **1,83** |

### Beispiel 4: Erfindungsgemäßes Verfahren Fig.11 - Herstellung von emissionsarmen Toluol-diisocyanat (TDI), Einsatzes von regenerativ hergestelltem Wasserstoff zur Beheizung der Brennkammer

Einem Reformerprozess (6) wurden 6,92 t/h Erdgas, mit einer Zusammensetzung von mind. 98% Methan und mind. 1% weiteren Nebenbestandteilen wie Ethan und höheren Kohlenwasserstoffen, 19,0 t/h Wasserdampf, sowie 3,76 t/h CO₂ aus der CO₂ Abtrenneinheit (8) eingebracht. Als Brenngas die Brennkammer des Heizelementes (61) wurde Wasserstoff (61d) sowie das Restgas (70a) aus der Restgasbehandlung (70) eingesetzt. Der Brennkammer des Heizelementes (61) wurden 1,05 t/h Wasserstoff (61d) sowie das 0,92 t/h Restgas (70a) aus der Restgasbehandlung bestehend aus 0,60 t/h CO, 0,26 t/h H₂ und 0,06 t/h Methan zugeführt. Der Reformerprozess wurde bei einer Temperatur von 950°C und bei einem Druck von 20 bar durchgeführt.

Aus dem Reformerprozess erhaltene Produktgasgemisch (20) überwiegend bestehend aus 12,0 t/h CO, 11,32 t/h H₂O, 3,76 t/h CO₂, 2,58 t/h H₂ und 0,06 t/h Methan wurde einer H₂O Abtrenneinheit (7) zugeführt, in der 11,32 t/h Wasser aus dem Gasgemisch (20) abgetrennt wurden. Das verbleibende Gasgemisch (21) aus der H₂O-Abtrennung (7) wurde einer CO₂ Abtrenneinheit (8) zugeführt, in der 3,76 t/h CO₂ abgetrennt und dem Reformerprozess (6) wieder zugeführt wurden. Die CO₂ Abtrennung erfolgt mittels Aminwäsche. Das von CO₂ befreite Gas (22) bestehend aus 12,0 t/h CO und 2,58 t/h H₂ sowie 0,06 t/h Methan wurde der H₂-CO Trenneinheit (9) zugeführt. Für die H₂-CO Trennung wurde eine sogenannte Cold-box eingesetzt, bei der das H₂-CO Gasgemisch abgekühlt und Wasserstoff und CO getrennt wurden. Das Restgas (9b) aus der H₂-CO-Trenneinheit (9) wurde der Restgasbehandlung (70) zugeführt. Das Restgas (9b) besteht aus 0,6 t/h CO, 2,58 t/h H₂ und 0,06 t/h CH₄. Aus der Restgasbehandlung (70) wurde ein Restgas (70a) mit 0,92 t/h bestehend aus 0,26 t/h H₂, 0,60 t/h CO und 0,06 t/h Methan abgetrennt und der Brennkammer des Heizelementes (61) zugeführt. Aus der Restgasbehandlung wurden 2,32 t/h H₂ abgetrennt und 1,05 t/h H₂ (61d) der Brennkammer des Heizelementes (61) sowie 1,27 t/h (70e) für die Hydrierung der Nitroverbindungen (32) eingesetzt.

Weiterhin wurden 0,81 t/h H₂ aus der Salzsäureelektrolyse (12a) sowie 0,36 t/h H₂ aus einer Wasserelektrolyse (5) der Hydrierung der Nitroaromaten (32) zugeführt wurde.

Aus der H₂-CO Trenneinheit (9) wurden 11,4 t/h CO der Phosgensynthese zugeführt.

18,72 t/h Toluol wurden in einer Dinitrotoluol-Herstellung (30) mit 25,65 t/h Salpetersäure, gerechnet als HNO₃, zu 37,04 t/h Dinitrotoluol umgesetzt. Das Dinitrotoluol wurde anschließend in einer Hydrierungsstufe (32) mit 2,44 t/h Wasserstoff aus der Restgasbehandlung, der Salzsäureelektrolyse und der Wasserelektrolyse umgesetzt. Dabei entstehen 24,63 t/h Toluol-Diamin welches anschließend in einer Isocyanat-Herstellung (2) mit Phosgen zu Isocyanat umgesetzt wurde.

Das Phosgen wurde in der Phosgen-Synthese (1) hergestellt aus 11,4 t/h CO und 28,90 t/h Chlor, welches aus einer Chlor-Herstellung (12) durch eine Salzsäureelektrolyse erzeugt wurde.

Aus der Phosgensynthese (1) wurden 40,3 t/h Phosgen entnommen und in einer Isocyanat-Herstellung (2) mit 24,63 t/h Toluol-Diamin zu 35,41 t/h Toluol-diisocyanat umgesetzt. Das erhaltene Toluol-diisocyanat kann in üblicher Weise mit Polyetherpolyolen oder Polyesterpolyolen in einer Polyurethan-Synthese zu Polyurethan-Material umgesetzt werden.

Das bei der Isocyanat-Herstellung anfallende HCl-Gas (25) mit einer Menge von 29,71 t/h wurde nach Reinigung über eine Tieftemperaturdestillation in Wasser absorbiert und die entstehende Salzsäure nochmals über Aktivkohle gereinigt und in einer Salzsäureelektrolyse zu 28,9 t/h Chlor und 0,81 t/h Wasserstoff umgesetzt.

Aus dem Reformer (6) kann Dampf mit einem Energieinhalt von 6,1 MWh/h erhalten werden. Hierzu wurde bei der Dampferzeugung zusätzlich Erdgas (13a) mit 0,32 t/h zugeführt.

| CO₂-Quelle | t/h CO2 |
|---|---|
| Brennkammer- Verbrennung Restgas 70a (aus CO und CH4 des Restgases) | 1,11 |
| Dampferzeugung - Verbrennung Erdgas 13a | 0,89 |
| **Gesamte CO₂ Emissionen** | **2,0** |

### Beispiel 5: Erfindungsgemäßes Verfahren Fig.12 - Herstellung von emissionsarmen Toluol-diisocyanat (TDI), Reformerprozess mit Zuführung von externem CO₂

In einem Reformerprozess (6) wurden 3,59 t/h Erdgas, mit einer Zusammensetzung von mind. 98% Methan und mind. 1% weiteren Nebenbestandteilen wie Ethan und höheren Kohlenwasserstoffen, 12,0 t/h Wasserdampf, sowie 10,22 t/h CO₂ (8a) aus der CO₂ Abtrenneinheit (8) sowie 8,95 t/h CO₂ (4a) aus externer Quelle (z.B. aus der Herstellung von Zement) eingebracht. Der Reformerprozess wurde bei einer Temperatur von 950°C und bei einem Druck von 20 bar durchgeführt.

Aus dem Reformerprozess erhaltenes Produktgasgemisch (20) überwiegend bestehend aus 12,0 t/h CO, 11,63 t/h H₂O, 10,22 t/h CO₂, 0,94 t/h H₂ und 0,01 t/h Methan wurde einer H₂O Abtrenneinheit (7) zugeführt, in der 11,63 t/h Wasser aus dem Gasgemisch (20) abgetrennt wurden. Das verbleibende Gasgemisch (21) aus der H₂O-Abtrennung (7) wurde einer CO₂ Abtrenneinheit (8) zugeführt, in der 10,22 t/h CO₂ abgetrennt (8a) und dem Reformerprozess (6) wieder zugeführt wurden. Die CO₂ Abtrennung erfolgte mittels Aminwäsche. Das von CO₂ befreite Gas (22) bestehend aus 11,9 t/h CO und 0,9 t/h H₂ sowie 0,01 t/h Methan wurde der H₂-CO Trenneinheit (9) zugeführt. Für die H₂-CO Trennung wurde eine Cold-box eingesetzt, bei der das H₂-CO Gasgemisch abgekühlt und Wasserstoff und CO getrennt wurden. Dabei wurde ein Restgas (tail gas) (9b) abgetrennt, welches aus H₂, CO und weiteren Nebenbestandteilen wie Methan bestand. Von den 1,45 t/h Restgas (9b) wurden 0,50 t/h in die Dampferzeugung (13) und 0,95 t/h in die Brennkammer des Reformers (61) geführt.

18,72 t/h Toluol wurden in einer Dinitrotoluol-Herstellung (30) mit 25,65 t/h Salpetersäure, (HNO₃) zu 37,04 t/h Dinitrotoluol umgesetzt. Das Dinitrotoluol wurde anschließend in einer Hydrierungsstufe (32) mit 2,44 t/h Wasserstoff umgesetzt, der aus einer Wasserelektrolyse (5) der Chlor-Herstellung (12) einer Salzsäureelektrolyse stammte. Dabei entstanden 24,63 t/h Toluol-Diamin welches anschließend in einer Isocyanat-Herstellung (2) mit Phosgen zu Isocyanat umgesetzt wurde.

Das Phosgen wurde in der Phosgen-Synthese (1) hergestellt aus 11,4 t/h CO und 28,90 t/h Chlor, welches aus einer Chlor-Herstellung (12) durch eine Salzsäureelektrolyse erzeugt wird.

Aus der Phosgensysnthese (1) wurden 40,3 t/h Phosgen entnommen und in einer Isocyanat-Herstellung (2) mit 24,63 t/h Toluol-Diamin zu 35,41 t/h Toluol-diisocyanat umgesetzt. Das erhaltene Toluol-diisocyanat kann in üblicher Weise mit Polyetherpolyolen oder Polyesterpolyolen in einer Polyurethan-Synthese zu Polyurethan-Material umgesetzt werden.

Das bei der Isocyanat-Herstellung anfallende HCl-Gas (25) mit einer Menge von 29,71 t/h wurde nach Reinigung über eine Tieftemperaturdestillation in Wasser absorbiert und die entstehende Salzsäure nochmals über Aktivkohle gereinigt und in einer Salzsäureelektrolyse zu 28,9 t/h Chlor und 0,81 t/h Wasserstoff umgesetzt. Der Wasserstoff aus der Salzsäureelektrolyse wurde mit 1,63 t/h H₂ aus einer Wasserelektrolyse für die Hydrierung der Nitroverbindungen (32) eingesetzt.

Aus dem Reformer (6) konnte Dampf mit einem Energieinhalt von 9,0 MWh/h erhalten werden.

| CO₂-Quelle | t/h CO₂ |
|---|---|
| Dampferzeugung - Verbrennung Restgas 70a | 0,01 |
| Brennkammer - Verbrennung Restgas 70a | 0,52 + 0,27 |
| CO₂ Gutschrift aus der Dampferzeugung | -0,53 |
| **Gesamte CO₂ Emissionen** | **0,27** |

## Patentansprüche

1. Verfahren für die Herstellung von Kohlenmonoxid aus Methan, Wasserdampf und CO₂ für die Darstellung von Phosgen zur Synthese von organischem Isocyanat, enthaltend zumindest die Schritte
Synthese von Kohlenmonoxid in einem Reformerprozess (6), worin Methan (6a) und Wasserdampf (3) unter Zusatz von mindestens CO₂ (8a),
unter Zuführung von Wärmeenergie bei einer Temperatur von mindestens 500°C zu Kohlenmonoxid-haltigem Produktgas umgesetzt wird,
Reinigung des aus vorgenannter Synthese erhaltenen Kohlenmonoxid-haltigen Produktgases, mindestens durch Abtrennung von CO₂ (8) und gegebenenfalls zusätzlich durch mindestens eine Abtrennung ausgewählt aus der Abtrennung von Wasser (7), der Abtrennung von Wasserstoff (9) oder einer Kombination davon, unter Erhalt von Kohlenmonoxid;
Bereitstellung von CO₂ (8a) für den besagten Zusatz zum vorgenannten Reformerprozess (6) zumindest aus besagter Abtrennung von CO₂ (8) des vorgenannten Reinigungsschritts,
mit der Maßgabe, dass
i) die zur Synthese von Kohlenmonoxid dem Reformerprozess (6) zugeführte Wärmeenergie durch mindestens eine Methode bereitgestellt wird, ausgewählt aus (i) der Verbrennung von Brennstoff, der mittels regenerativer Energie erzeugten Wasserstoff enthält, (ii) der Verbrennung von Brennstoff, der Methan aus biologischer Quelle enthält, (iii) Umwandlung von aus regenerativer Energie erzeugter, elektrischer Energie (6c) in Wärme; oder
ii) für die Bereitgestellung des der Synthese des vorgenannten Reformerprozesses (6) zugesetzten CO₂ zusätzlich CO₂ (4a) aus einer weiteren CO₂-Quelle (60) herangezogen wird; oder
iii) eine Kombination der vorgenannten Möglichkeiten i) und ii) gewählt wird.

2. Verfahren gemäß Anspruch 1, **dadurch gekennzeichnet, dass** als Methan-Quelle zur Bereitstellung des Methans (6a) für den Reformerprozess (6) Methan aus fossiler Quelle, Methan aus biologischer Quelle oder Gemische daraus, insbesondere Methan aus biologischer Quelle, dient.

3. Verfahren nach Anspruch 1 oder 2, **dadurch gekennzeichnet, dass** die als regenerative Energie wahlweise Windkraft, Sonnenenergie, Wasserkraft oder Mischungen daraus, eingesetzt wird.

4. Verfahren gemäß einem der Ansprüche 1 bis 3, **dadurch gekennzeichnet, dass** der für die Verbrennung von Wasserstoff-haltigem Brennstoff eingesetzte Wasserstoff durch mindestens ein Elektrolyseverfahren bereitgestellt wird, ausgewählt aus der Wasserelektrolyse (5) von Wasser (5c) zu Wasserstoff (5a) und Sauerstoff (5b), der Chlor-Alkali-Elektrolyse, der Salzsäure-Elektrolyse, wobei die für das Elektrolyseverfahren genutzte elektrische Energie mittels regenerativer Energie (15) erzeugt wird.

5. Verfahren gemäß einem der vorangehenden Ansprüche, **dadurch gekennzeichnet, dass** die Synthese im Reformerprozess (6) bei einer Temperatur von mindestens 700°C, bevorzugt in einem Bereich von 800 bis 1100 °C, besonders bevorzugt in einem Bereich von 850 bis 1000°C, abläuft.

6. Verfahren gemäß einem der vorangehenden Ansprüche, **dadurch gekennzeichnet, dass** die Synthese im Reformerprozess (6) bei einem absoluten Druck in einem Bereich von 10 bis 40 bar, bevorzugt von 15 bis 35 bar, besonders bevorzugt 18 bis 25 bar, abläuft.

7. Verfahren gemäß einem der vorangehenden Ansprüche, **dadurch gekennzeichnet, dass** die Synthese im Reformerprozess (6) unter Einsatz eines an einer festen Phase geträgerten Katalysators stattfindet.

8. Verfahren gemäß einem der vorangehenden Ansprüche, **dadurch gekennzeichnet, dass** der Reformerprozess (6) mindestens zwei Schritte umfasst
a) Umsetzung von zumindest Methan (6a) und Wasserdampf zu einem Gemisch, mindestens enthaltend Kohlenmonoxid und Wasserstoff
b) Umsetzung zumindest des besagten Gemisches aus (a) unter Zusatz von CO₂ zu Kohlenmonoxid-haltigem Produktgas.

9. Verfahren gemäß einem der vorangehenden Ansprüche, **dadurch gekennzeichnet, dass** die besagte Abtrennung von Wasser (7) erfolgt und bevorzugt das dabei abgetrennte Wasser (7a) in eine Wasserelektrolyse (5) rezykliert wird.

10. Verfahren gemäß einem der vorangehenden Ansprüche, **dadurch gekennzeichnet, dass** nach der durchgeführten Abtrennung von Wasserstoff (9),insbesondere unter Verwendung einer Coldbox, der aus dem Restgas (9b) nach weiterer Restgasbehandlung (70) hervorgehende Wasserstoff (70b) in eine Hydrierung von organischer Nitroverbindung (30) zur Herstellung von organischem Amin als Rohstoff für organisches Isocyanat eingespeist oder das Restgas der H₂-CO Trennung (9b) als Brennstoff zur Bereitstellung von Wärmeenergie des Reformerprozesses (6) in der Brennkammer des Heizelementes oder zur Wasserdampf-Erzeugung genutzt und/oder das nach weiterer Restgasbehandlung (70) erhaltene Restgas der Restgasbehandlung (70a) als Brennstoff zur Bereitstellung von Wärmeenergie des Reformerprozesses in der Brennkammer des Heizelementes (61) oder als erhaltenes Restgas der Restgasbehandlung (70c) zur Wasserdampf-Erzeugung genutzt.

11. Verfahren gemäß einem der vorangehenden Ansprüche, **dadurch gekennzeichnet, dass** eine Synthese (1) von Phosgen (1a) aus zumindest Kohlenmonoxid (9a) und Chlor (12b) erfolgt, und bevorzugt anschließend eine Umsetzung (2) von Phosgen (1a) mit mindestens einem organischen Amin (23) zu mindestens einer organischen Isocyanat-Verbindung (24) und Chlorwasserstoff (25) erfolgt.

12. Verfahren nach Anspruch 11, **dadurch gekennzeichnet, dass** eine Umsetzung (2) von Phosgen (1a) mit mindestens einem organischen Amin (23) zu mindestens einer organischen Isocyanat-Verbindung (24) und Chlorwasserstoff (25) erfolgt und die Abtrennung und Reinigung des in der Isocyanat-Herstellung (2) gebildeten Chlorwasserstoffs (25) und eine anschließende oxidative Umsetzung (12) des Chlorwasserstoffs, ausgewählt aus
- einer Umsetzung von Chlorwasserstoff in einer thermokatalytischen Gasphasenoxidation mit Sauerstoff zu Chlor und Wasser unter ggf. Nutzung von Sauerstoff aus der Wasserelektrolyse (5),
- einer Umsetzung von Chlorwasserstoff mit Wasser zu Salzsäure und anschließender elektrochemischer Oxidation der Salzsäure zu Chlor und ggf. Wasserstoff,
erfolgt und an die Umsetzung (12) eine Zuführung des gebildeten Chlors (12b) in die Phosgensynthese (1) anschließt, gegebenenfalls unter Zuführung einer zusätzlichen Menge Chlor in die Phosgensynthese (1), die aus einer Chloralkali-Elektrolyse stammt.

13. Verfahren gemäß einem der vorangehenden Ansprüche, **dadurch gekennzeichnet, dass** mindestens 80 % der für das gesamte Verfahren eingesetzten Energie mittels aus regenerativer Energie erzeugten elektrischen Stroms (6c) durchgeführt wird, insbesondere elektrischen Stroms (6c) wahlweise erhalten durch Nutzung von Windkraft, Sonnenenergie oder Wasserkraft.

14. Vorrichtung für die Synthese von Kohlenmonoxid unter Bereitstellung von Kohlenmonoxid für die Herstellung von Phosgen für die Synthese von organischem Isocyanat umfasst mindestens folgende Vorrichtungsteile
- mindestens einen Reformer, enthaltend mindestens einen Reaktionsraum, mindestens ein Heizelement als eine Vorrichtung zur Zuführung von Wärmeenergie zum Reaktionsraum, sowie mindestens einen Einlass für die Einbringung von Methan, Wasserdampf und CO₂ in den Reaktionsraum und mindestens einen Auslass für das Kohlenmonoxid-haltige Produktgas aus dem Reformer;
- mindestens eine Vorrichtung zur Abtrennung von Wasser, die zumindest einen Einlass aufweist, der in Fluidverbindung mit dem Auslass für das Kohlenmonoxid-haltige Produktgas aus dem Reformer steht und mindestens einen Auslass für das abgetrennte Wasser und mindestens einen Auslass für das getrocknete, Kohlenmonoxid-haltigen Produktgas;
- mindestens eine Vorrichtung zur Abtrennung von CO₂, die zumindest einen Einlass aufweist, der in Fluidverbindung mit dem Auslass für das getrocknete, Kohlenmonoxid-haltige Produktgas aus der Vorrichtung zur Abtrennung von Wasser steht und mindestens einen Auslass für das abgetrennte CO₂, der in Fluidverbindung mit einem Einlass für CO₂ des Reformers steht und mindestens einen Auslass für das vorgereinigte, Kohlenmonoxid-haltige Produktgas,;
- mindestens eine Vorrichtung zur Trennung von H₂-CO, die zumindest einen Einlass aufweist, der in Fluidverbindung mit dem Auslass für das vorgereinigte, Kohlenmonoxid-haltige Produktgas aus der Vorrichtung zur Abtrennung von CO₂ steht und mindestens einen Auslass für das Kohlenmonoxid und mindestens einen Auslass für das Restgas der H₂-CO Trennung;
- mindestens eine Vorrichtung zur Herstellung von Phosgen, die mindestens einen Einlass für Kohlenmonoxid aufweist, der in Fluidverbindung mit dem für das Kohlenmonoxid vorgesehenen Auslass der Vorrichtung zur Trennung von H₂-CO in Fluidverbindung steht, sowie mindestens einen Einlass für Chlor und mindestens einen Auslass für Phosgen enthält;
- mindestens eine Vorrichtung zur Herstellung von Chlor, die mindestens einen Einlass für Chlorwasserstoff aufweist, sowie mindestens einen Auslass für Wasserstoff und mindestens einen Auslass für Chlor enthält, der mit einem Einlass für Chlor von der Vorrichtung zur Herstellung von Phosgen in Fluidverbindung steht;
mit der Maßgabe, dass
i) das Heizelement des Reformers in Fluidverbindung mit mindestens einer Quelle, ausgewählt aus einer Quelle für aus regenerativer Energie bereitgestelltem Wasserstoff als Brennstoff, einer Quelle für aus biologischer Quelle bereitgestelltem Methan als Brennstoff, steht oder das Heizelement an eine Quelle zur Bereitstellung von elektrischer Energie aus regenerativer Energie für die Umwandlung in Wärmeenergie angeschlossen ist; und/oder
ii) ein Einlass des Reaktors in Fluidverbindung mit einer weiteren CO₂-Quelle zur Einbringung von zusätzlichem CO₂ in den Reaktionsraum steht, herangezogen wird.

15. Verwendung einer Vorrichtung, die für die Synthese von Kohlenmonoxid in einem Reformerprozess ausgestaltet ist, worin Methan mit Wasserdampf unter Zusatz von mindestens CO₂, unter Zuführung von Wärmeenergie bei einer Temperatur von mindestens 500°C zu Kohlenmonoxid-haltigem Produktgas umgesetzt wird, und
die für die Reinigung des aus vorgenannter Synthese erhaltenen Kohlenmonoxid-haltigen Produktgases ausgestaltet ist, mindestens durch Abtrennung von Wasser, durch Abtrennung von CO₂, durch Abtrennung von Wasserstoff oder durch eine Kombination von mindestens zwei der vorgenannten Abtrennungen in jeweils eine entsprechenden Abtrenneinheit, unter Erhalt von Kohlenmonoxid,
zur Bereitstellung von Kohlenmonoxid für die Herstellung von organischem Isocyanat, nach einem Verfahren, umfassend zumindest die Schritte
Synthese von Phosgen aus zumindest Kohlenmonoxid und Chlor,
Umsetzung von Phosgen mit mindestens einem organischen Amin zu mindestens einer organischen Isocyanat-Verbindung und Chlorwasserstoff.

16. Verwendung nach Anspruch 15, wobei die Vorrichtung eine Vorrichtung gemäß Anspruch 14 ist.
